# EUROPEAN PATENT SPECIFICATION

(11) **EP 2 480 221 B1**
(45) Date of publication and mention of the grant of the patent: **04.04.2018**
(21) Application number: 10819449.9
(22) Date of filing: 23.09.2010
(51) Int. Cl.: A61K 9/68, A61P 25/34, B29C 71/04

(54) **PROCESS FOR THE MANUFACTURE OF NICOTINE-COMPRISING CHEWING GUM AND NICOTINE-COMPRISING CHEWING GUM MANUFACTURED ACCORDING TO SAID PROCESS**
VERFAHREN ZUR HERSTELLUNG EINES NIKOTINHALTIGEN KAUGUMMIS UND IN DIESEM VERFAHREN HERGESTELLTES NIKOTINHALTIGES KAUGUMMI
PROCÉDÉ DE PRODUCTION D'UNE GOMME À MÂCHER CONTENANT DE LA NICOTINE ET GOMME À MÂCHER PRODUITE CONFORMÉMENT AU DIT PROCÉDÉ

(30) Priority: 22.09.2010 US 887593; 28.10.2009 US 255582 P; 24.09.2009 US 245315 P
(43) Date of publication of application: 01.08.2012
(73) Proprietor: Johnson & Johnson Consumer Inc., Skillman, NJ 08558 (US); Nicklasson, Fredrik, 237 32 Bjärred (SE)
(72) Inventor: NICKLASSON, Fredrik, S-237 31 Bjärred (SE); HUGERTH, Andreas, S-237 33 Bjärred (SE); LINDELL, Katarina, S-241 93 Eslöv (SE); HENDENSTROM, John, S-256 54 Ramlösa (SE); KOLL, Gregory, E., Hillsborough NJ 08844 (US); SOWDEN, Harry, S., Glenside PA 19038 (US); LUBER, Joseph, R., Quakertown, PA 18951 (US); KRIKSUNOV, Leo, B., Ithaca NY 14850 (US); BUNICK, Frank, J., Randolph NJ 07869 (US); CHEN, Jen-chi, Morrisville 19067 (US); OLSSON, Roland, S-253 Gantofta (SE); SZYMCZAK, Christopher, E., Marlton NJ 08053 (US)
(74) Representative: Kirsch, Susan Edith
(86) International application number: PCT/US2010/049974
(87) International publication number: WO 2011/038104

(56) References cited:
- WO-A1-2009/080022
- WO-A2-2009/037319

## Description

### Background of the Invention

Advantages with radio frequency (RF)-technology in comparison with other manufacturing technologies.

The RF-technology offers a possibility of manufacturing nicotine-comprising chewing gums that may result in the following advantages over other gum manufacturing methods, such as (i) improved mouth-feel with RF-treated gums being less crumbly than directly compressed gums; (ii) formulations with higher gum base content than is currently possible with direct compression of gum base material; (iii) incorporating encapsulated ingredients, such as flavors, buffers and other additives that would be broken if manufactured using direct compression or mixing, rolling and scoring, (iv) including flakes of polyols and/or sugar in the formulation to provide a crispy/crunchy mouth-feel, and/or (v) Other gum shapes possible in comparison with manufacturing using rolling-and-scoring.

RF energy is one type of electromagnetic energy (EM energy). As will be seen below also other types of EM energy may be useful in the present invention.

WO-A-2009/037319 discloses use of a nicotine-cellulose combination, maltitol and a gum base for the preparation of a chewing gum composition for achieving a fast onset of nicotine effect after initiation of chewing the chewing gum composition by a subject. The chewing gum composition is preferably prepared by direct compression and it does not disintegrate during chewing.

WO-A-2009/080022 discloses a compressed chewing gum tablet comprising one or more pharmaceutically active ingredients and one or more enhancers.

### Summary of the Invention

The present invention features a process as defined in appended claim 1 for making a nicotine-comprising chewing gum by (i) dispensing a powder portion from a gum-base-comprising powder , (ii) optionally shaping said powder portion into a powder aggregate, and (iii) applying sufficient electromagnetic energy (EM energy) to said powder portion or said powder aggregate to transform said powder portion or said powder aggregate into said chewing gum, whereby said EM energy is RadioFrequency (RF) energy, MicroWave (MW) energy, InfraRed (IR) energy, UltraViolet (UV) energy or combinations thereof, preferably Radio Frequency (RF) energy, the combination of RF energy and IR energy, the combination of RF energy and MW energy, and the combination of RF energy, IR energy and MW energy.

In one embodiment the EM energy has a frequency such that it is non-ionizing, meaning below about 1000 THz.

Also disclosed but not claimed is a nicotine-comprising chewing gum made by such process.

Said nicotine-comprising chewing gum may further comprise one or more deposits.

Said nicotine-comprising chewing gum may be coated.

Other aspects, as well as features and advantages of the present invention will be apparent from the detailed description of the invention and from the claims.

### Brief Description of the Figures

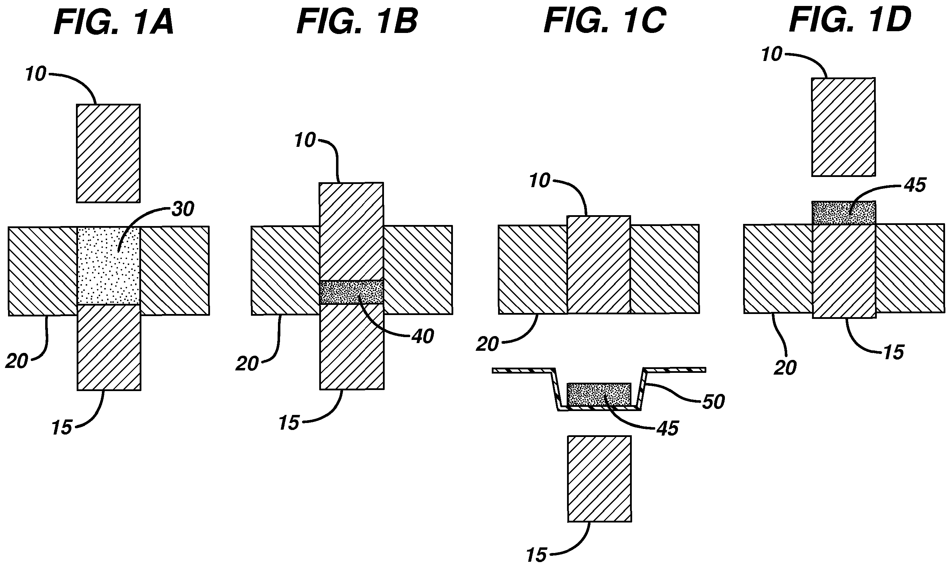

FIG. 1A is a side view of an embodiment of the invention showing gum-base-comprising powder 30 dispensed into a die 20.
FIG. 1B is a side view of an embodiment of the invention showing a powder portion 40 being densified between an upper punch 10 and a lower punch 15 thereby being shaped into a powder aggregate.
FIG. 1C is a side view of an embodiment of the invention showing chewing gum 45 pushed by the upper punch 10 from the die 20 into blister 50.
FIG. 1D is a side view of an embodiment of the invention showing chewing gum 45 pushed from the die 20 by the lower punch 15.

### Detailed Description of the Invention

It is believed that one skilled in the art can, based upon the description herein, utilize the present invention to its fullest extent.

Unless defined otherwise, all technical and scientific terms used herein have the same meaning as commonly understood by one of ordinary skill in the art to which the invention belongs. As used herein, all percentages are by weight unless otherwise specified.

### Gum Base

The gum base may be of any conventional gum base known in the art. For example it may be of natural or synthetic origin. Natural gum bases include, but are not limited to, chicle, jelutong-, lechi de caspi-, soh-, siak-, katiau-, sorwa-, balata-, pendare-, malaya-, and peach gums; natural cautchouc; and natural resins such as dammar and mastix. Synthetic gum bases may comprise elastomers (polymers, masticating substances), plasticizer (resin, elastomers, solvent, hydrophobic resin), filler (texturizer, water-insoluble adjuvant), softener (fat), emulsifier, wax, antioxidant, and anti-tacking agents (vinyl polymer hydrophilic resin). Additionally other examples of gum bases are gums including agar, alginate, Arabic gum, carob gum, carrageenan, ghatti gum, guar gum, karaya gum, pectin, tragacanth, locust bean gum, gellan gum and xanthan gum.

Also known in the art are gum bases that are designed to be utilized in the manufacture of chewing gum by method of direct compression (DC) in a standard tablet press. These DC-gum bases are co-processed materials, where conventional gum base is mixed with other excipients, such as polyols and anti-caking agents, and the powder mix is then processed to form composite particles comprising the ingredients of said mix. Several grades of DC-gum bases are commercially available, under trade names such as HiG PWD-03 (Cafosa Corporation, Spain). The upper limit of conventional gum base content in DC-gum base is about 35% (w/w). Higher contents of conventional gum base in the DC-gum base is not feasible due to excessive sticking of DC-gum base to the dies, punches and other surfaces of a tablet press.

In one embodiment, the weight percentage of gum base in the gum-base comprising powder is from about 10% to about 80%, preferably from about 20% to about 80 %, more preferably from about 30% to about 80%, and even more preferably from about 40% to about 70%.

The gum-base comprising powder has an average particle size of less than 2000 microns, preferably less than 1000 microns, and even more preferably less than 500 microns and most preferably less than 300 microns.

### Nicotine

The gum base-comprising powder or powder blend and/or the one or more deposits comprise(s) nicotine in any form.

The nicotine may be present in its free base form.

Numerous nicotine salts are known and may be used. Examples include, but are not limited to, formic (2:1), acetic (3:1), propionic (3:1), butyric (3:1), 2-methylbutyric (3:1), 3-methylbutynic (3:1), valeric (3:1), lauric (3:1), palmitic (3:1), tartaric (1:1) and (2:1), citric (2:1), malic (2:1), oxalic (2:1), benzoic (1:1), gentisic (1:1), gallic (1:1), phenylacetic (3:1), salicylic (1:1), phthalic (1:1), picric (2:1), sulfosalicylic (1:1), tannic (1:5), pectic (1:3), alginic (1:2), hydrochloric (2:1), chloroplatinic (1:1), silcotungstic (1:1), pyruvic (2:1), glutamic (1:1), and aspartic (1:1) salts of nicotine.

In one embodiment, the nicotine in any form is bound to a resin (e.g., a polyacrylate resin), zeolite, or cellulose or starch microsphere. Examples of cation exchange resins include, but are not limited to, Amberlite IRC 50 (Rohm & Haas), Amberlite IRP 64 (Rohm & Haas), Amberlite IRP 64M (Rohm & Haas), BIO-REX 70 (BIO-RAD Lab.), Amberlite IR 118 (Rohm & Haas), Amberlite IRP 69 (Rohm & Haas), Amberlite IRP 69M (Rohm & Haas), BIO-REX 40 (BIO-RAD Lab.), Amberlite IR 120 (Rohm & Haas), Dowex 50 (Dow Chemical), Dowex 50W (Dow Chemical), Duolite C 25 (Chemical Process Co.), Lewatit S 100 (Farbenfabriken Bayer), Ionac C 240 (Ionac Chem.), Wofatit KP S 200 (I.G. Farben Wolfen), Amberlyst 15 (Rohm & Haas), Duolite C-3 (Chemical Process), Duolite C-10 (Chemical Process), Lewatit KS (Farbenfabriken Bayer), Zerolit 215 (The Permutit Co.), Duolite ES-62 (Chemical Process), BIO-REX 63 (BIO-RAD Lab.), Duolite ES-63 (Chemical Process), Duolite ES-65 (Chemical Process), Ohelex 100 (BIO-RAD Lab.), Dow Chelating Resin A-1 (Dow Chemical Company), Purolite C115HMR (Purolite International Ltd.), CM Sephadex C-25 (Pharmacia Fine Chemicals), SE Sephadex C-25 (Pharmacia Fine Chemicals), Viscarin GP-109NF Lambda-carrageenan FMC Biopolymer or any other anionic polyelectrolyte.

In one another embodiment, the nicotine in any form is in the form of an inclusion complex with a cyclodextrin, which may include cyclodextrin complexation, such as complexation of the active pharmaceutically compound with cyclodextrin where preferably the cyclodextrin used is chosen among α-, β- and γ-cyclodextrin, the hydroxypropyl derivatives of α-, β- and γ-cyclodextrin, sulfoalkylether cyclodextrins such as sulfobutylether β-cyclodextrin, alkylated cyclodextrins such as the randomly methylated β-cyclodextrin, and various branched cyclodextrins such as glucosyl- and maltosyl-β-cyclodextrin.

In one embodiment, the nicotine is dosed in the chewing gum to provide the person with a dose to achieve an effect, e.g. to provide a sense of smoking satisfaction without smoking and/or to reduce of the urge to smoke or use tobacco. This amount may, of course, vary from person to person.

In one embodiment, the chewing gum comprises nicotine in an amount of from about 0.05 mg to about 12 mg calculated as the free base form of nicotine per chewing gum, such as from about 0.2 mg to about 8 mg, more preferably from about 0.5mg to about 6 mg, and even more preferably from about 1 mg to about 5 mg. This may in different embodiments include 0.05, 0.5, 1, 1.5, 2, 3, 4, 4.5, 5, 6, 7, 8, 9, 10 or 12 mg calculated as the free base form of nicotine per chewing gum.

Hereby the nicotine may be present in different parts of the chewing gum. If one or more deposits are present, said deposits may comprise nicotine in any form. The nicotine may be present in the chewing gum in more than one form, e.g. as resinate as well as hydrogen tartrate salt.

The nicotine may be present in different forms in different parts of the chewing gum.

### Buffering Agent

In one embodiment, the chewing gum further comprises one or more buffering agents. In one embodiment, the chewing gum is buffered such that upon administration of the gum, the pH of the saliva is transiently increased from about 0.2 to about 4 pH units, preferably from abot 0.4 to about 2 pH units. The buffering is designed so as to achieve a transient buffering of the saliva of a subject during mastication of the chewing gum. As the change is transient, the pH will return to its normal value after a certain period of time.

Examples of buffering agents include, but are not limited to, carbonates including carbonate, bicarbonate or sesquicarbonate, glycinate, phosphate, glycerophosphate or citrate of an alkali metal, such as potassium or sodium, or ammonium such as trisodium or tripotassium citrate, trisodium phosphate, disodium hydrogen phosphate, tripotassium phosphate, dipotassium hydrogen phosphate, calcium hydroxide, sodium glycinate and trometamol (TRIS). Alkali metal carbonates, glycinates and phosphates are preferred buffering agents.

The one or more buffering agents may to some extent be microencapsulated or otherwise coated as granules with polymers and/or lipids being less soluble in saliva than is the one or more buffering agents. Such microencapsulation controls the dissolution rate whereby is extended the time frame of the buffering effect.

In order to increase the buffering capacity still further without correspondingly increasing the pH, one may in specific embodiments use a second or auxiliary buffering agent to the first buffering agent, such as e.g., sodium or potassium bicarbonate buffers. The second or auxiliary buffering agent may be selected from the group consisting of alkali metal bicarbonates that are preferred for this purpose. Thus, further embodiments of the invention may comprise a mixture of an alkali metal carbonate or phosphate and alkali metal bicarbonate.

Hereby the buffering agent may be present in different parts of the chewing gum. If one or more deposits are present, said deposits may comprise buffering agents. The buffering agent may be present in the chewing gum in more than one form, e.g. as sodium carbonate as well as trometamol.

The amount of the buffering agent or agents in the chewing gum composition is preferably sufficient in the specific embodiments to raise the pH of the saliva to above 7, as specified above, to transiently maintain the pH of the saliva in the oral cavity above 7, e.g., pH 7-10.

As seen above the nicotine may be administered in different forms. The amount of buffer required to achieve said increase in pH with the different nicotine forms is readily calculated by the skilled man in the art. The extent and duration of the increase in pH is dependent on type and amount of the buffering agent(s) used as well as where the buffer is distributed in the chewing gum.

### Further Excipients

As discussed above, a nicotine-comprising chewing gum is manufactured by (i) dispensing a powder portion from a gum-base-comprising powder , (ii) optionally shaping said powder portion into a powder aggregate, and (iii) applying sufficient electromagnetic energy (EM energy) to said powder portion or said powder aggregate to transform said powder portion or said powder aggregate into said chewing gum, whereby said EM energy is Radio Frequency (RF) energy, MicroWave (MW) energy , InfraRed (IR) energy or UltraViolet (UV) energy or combinations thereof, preferably Radio Frequency (RF) energy, the combination of RF energy and IR energy, the combination of RF energy and MW energy, and the combination of RF energy, IR energy and MW energy.

Optionally may be added further excipients. Examples of such excipients include, but are not limited to, softeners, fillers, thickening agents, emulsifiers, glidants, lubricants, sweeteners, flavors and aromatics, enhancers, coloring agents and preservatives and mixtures thereof.

Examples of fillers include, but are not limited to, polydextrose, hydrogenated starch hydrosylate and corn starch.

Examples of lubricants include, but are not limited to, long chain fatty acids and their salts, such as magnesium stearate and stearic acid, talc, glycerides waxes, and mixtures thereof.

Examples of glidants include, but are not limited to, colloidal silicon dioxide.

Examples of sweeteners include, but are not limited to, synthetic or natural sugars; artificial sweeteners such as saccharin, sodium saccharin, aspartame, acesulfame, thaumatin, glycyrrhizin, sucralose, dihydrochalcone, alitame, miraculin, monellin, and stevside; sugar alcohols such as sorbitol, mannitol, glycerol, lactitol, malitol, and xylitol; sugars extracted from sugar cane and sugar beet (sucrose), dextrose (also called glucose), hydrogenated starch hydrosylate, starch, maltodextrin, fructose (also called laevulose), and lactose (also called milk sugar); isomalt, salts thereof, and mixtures thereof.

Examples of flavors and aromatics include, but are not limited to, essential oils including distillations, solvent extractions, or cold expressions of chopped flowers, leaves, peel or pulped whole fruit comprising mixtures of alcohols, esters, aldehydes and lactones; essences including either diluted solutions of essential oils, or mixtures of synthetic chemicals blended to match the natural flavor of the fruit (e.g., strawberry, raspberry and black currant); artificial and natural flavors of brews and liquors, e.g., cognac, whisky, rum, gin, sherry, port, and wine; tobacco, coffee, tea, cocoa, and mint; fruit juices including expelled juice from washed, scrubbed fruits such as lemon, orange, and lime; spear mint, pepper mint, wintergreen, cinnamon, cacoe/cocoa, vanilla, liquorice, menthol, eucalyptus, aniseeds nuts (e.g., peanuts, coconuts, hazelnuts, chestnuts, walnuts, colanuts), almonds, raisins; and powder, flour, or vegetable material parts including tobacco plant parts, e.g., genus Nicotiana, in amounts not contributing significantly to the level of nicotine, and ginger. Suitable flavors and aromatics may be used in liquid, semisolid or solid form, such as sorbed to a carrier in powder form.

Examples of coloring agents include, but are not limited to, dyes being approved as a food additive.

In one embodiment one or more of the excipients ingredients is present in encapsulated form and/or as flakes or part of flakes and/or fracture sensitive formats.

Some of the captioned further excipients may be present in different and/or multiple capacities.

### Optional Sharing of Powder Portion into Powder Aggregate

The gum base and excipients, and optionally the nicotine in any form, such as discussed above, are mixed by any suitable method known in the art to form a powder or a powder blend. The powder or powder blend is then dispensed into separate powder portions, each powder portion comprising an amount of powder or powder blend suitable for a chewing gum. At this stage, the shape of the final chewing gum can be set by dispensing said powder portion into a pre-shaped mold, die, other cavity or other shape-forming means. In order to facilitate shaping, the powder portion may optionally be densified by tamping, compression, compaction, de-aeration, vacuum-forming, slugging, granulation, vibration or other suitable method. The shaped, optionally densified powder aggregate may then be transformed into chewing gum by the application of electromagnetic energy (EM energy), whereby said EM energy is Radio Frequency (RF) energy, MicroWave (MW) energy , InfraRed (IR) energy or UltraViolet (UV) energy or combinations thereof, preferably Radio Frequency (RF) energy, the combination of RF energy and IR energy, the combination of RF energy and MW energy, and the combination of RF energy, IR energy and MW energy. Optionally one or more deposits may be added to the powder portion, the powder aggregate or the chewing gum.

The type of EM energy, or optionally the mixture of EM energy types, which is most useful in the specific situation depends on the properties of the components making up the powder, the powder blend and/or the optional deposits. Such properties include e g the frequency/ies at which the electromagnetic interaction is optimal.

The person skilled in the art is knowledgeable about useful methods for assessing the degree of interaction with EM energy for compounds being of interest for incorporation into the chewing gums of the present invention. It should be noted that certain compounds do not interact, or interact very weakly, with any kind of EM energy, or interact only in non-useful frequency ranges.

The energy required per weight unit of the powder or the powder blend for transforming said powder or powder blend into a chewing gum is also depending on the electromagnetic interaction properties of the components making up the powder, powder blend and optional deposits. The skilled person is able to calculate or assess the energy amount required for obtaining the chewing gum.

It should be noted that choice of frequency for the EM energy is very important. For example may a certain frequency result in a very short time of manufacture, while at the same time quality of the resulting chewing gum will be unsatisfactory.

When the excipients have very different electromagnetic interaction properties it may be useful to use combinations of different EM energies. The respective frequencies and powers for said EM energies may be optimized through testing according to principles known to the person skilled in the art.

As is understood from the captioned disclosure optimizing application of the RF energy requires the taking into account of different parameters, such as choice electromagnetic frequency in relation to e g degree of electromagnetic interaction, industrial standards and effects on other objects than the product being treated, power of the RF apparatus, time for applying the RF energy, absorbed energy per weight unit of the product being treated, coefficient of utilization and batch size. The captioned reasoning applies *mutatis mutandis* to other types of EM energy.

In certain embodiments EM energy may be combined with thermal energy and/or mechanical energy.

In one embodiment, the powder portion is shaped into a powder aggregate using e.g. a punch and die apparatus. In one embodiment the powder or powder blend is fed into a die of an apparatus that applies pressure to shape a powder aggregate. Any suitable apparatus may be used, including, but not limited to, a conventional unitary or rotary tablet press such as those commercially available from Fette America Inc., Rockaway, N.J. or Manesty Machines LTD, Liverpool, UK. In one embodiment, the powder aggregate is treated with RF energy within the tablet press. In another embodiment, said powder aggregate is treated with RF energy after having been removed from the tablet press.

In one embodiment, as shown in FIG. 1A, a powder portion 30 is dispensed from a gum-base-comprising powder into a die 20, where the powder portion 30 is either gravity fed or mechanically fed from a feeder (not shown) of a rotary tablet press, and the die rotates as part of a die table from the filling position (FIG 1A) to a densification position (FIG 1B). At the densification position (FIG. 1B), the powder portion 30 is densified between an upper punch 10 and a lower punch 15 to shape a powder aggregate 40. The resulting powder aggregate 40 is then exposed to RF energy to form the chewing gum 45. In one embodiment as shown in FIG 1C, the chewing gum 45 is pushed by the upper punch 10 from the die 20 into a blister 50 used to package the chewing gum 45. In an alternative embodiment shown in FIG 1D, the chewing gum 45 is pushed from the die 20 by the lower punch 15 and guided to an ejection chute by a stationary "take-off" bar (not shown).

In one embodiment, the densification step occurs in an indexed manner, where one set of powder portions are densified simultaneously, before rotating to another indexing station. In one embodiment, the densification step occurs at a single indexing station and the application of RF energy occurs at a separate indexing station. In another embodiment, a third indexing station is present wherein the ejection of the chewing gum or multiple chewing gums occurs, wherein the lower punch is raised up through and up to the surface of the die. In another embodiment the densification step is performed through the addition of air pressure or hydraulic cylinder to the top of the upper punches. In one embodiment multiple chewing gums are ejected simultaneously and separated from the surface of the indexing station and removed via a take-off bar.

In another embodiment, the powder portion may be shaped by methods and apparatus described in United States Patent Application Publication No. 20040156902. Specifically, the powder aggregate is shaped using a rotary compression module including a fill zone, insertion zone, compression zone, ejection zone, and purge zone in a single apparatus having a double row die construction. The dies of the compression module may then be filled using the assistance of a vacuum, with filters located in or near each die. The purge zone of the compression module includes an optional powder blend recovery system to recover excess powder blend from the filters and return the powder blend to the dies. In one embodiment the die table is constructed of non-conductive material. The transformation of the powder portion and/or the powder aggregate into a chewing gum may be obtained by sintering and/or fusing and/or melting and/or mechanical interlocking.

In another and preferred embodiment the gum-base-comprising powder portion may be dispensed on top of a deposit such as, but not limited to, a directly compressed tablet, a hard-boiled lozenge or a jelly gum, whereby such a deposit may interact very weakly, or not at all, with RF energy.

The chewing gum may have one of a variety of different shapes. For example, it may be shaped as a parallelepiped, a three-dimensional representation of a spinnaker, a crescent, a hamburger, a disc, a heart, a polygon, a hexaflexagon, a circular object, an oval object, an oblong object, a polyhedron, such as a cube, a pyramid, a prism, a triangle, or the like; a space figure with some non-flat faces, such as a cone, a truncated cone, a cylinder, a sphere, a capsule-shaped object, a torus, or the like, whereby the chewing gum optionally has one or more major faces

In one embodiment a vibratory step is utilized (e.g., added after dispensing of the powder portion but prior to the RF treatment step, in order to shape and densify the powder portion into a powder aggregate). In one embodiment a vibration with the frequency from about 1 Hz to about 50 KHz is added with amplitude from 1 micron to 5 mm peak-to-peak is utilized to shape and densify the powder portion into a powder aggregate.

In one embodiment, a lubricant is added to the cavity prior to the dispensing of the powder portion. This lubricant may be a liquid or solid. Suitable lubricants include, but are not limited to solid lubricants such as magnesium stearate, starch, calcium stearate, aluminum stearate and stearic acid; or liquid lubricants such as but not limited to simethicone, lecithin, vegetable oil, olive oil, or mineral oil. In certain embodiments, the lubricant is added at a percentage by weight of the chewing gum product of less than 5 percent, e.g. less than 2 percent, e.g. less than 0.5 percent. In one embodiment, the chewing gum product is substantially free of a hydrophobic lubricant. Hydrophobic lubricants include, but are not limited to, magnesium stearate, calcium stearate and aluminum stearate.

### Radiofrequency and Other Electromagnetic Treatment to Form Chewing Gum

Radiofrequency (RF) energy is used to transform the gum-base-comprising powder portion or optional powder aggregate into a chewing gum. RF frequency is an electromagnetic energy within the range of from about 1 MHz to about 300 MHz. RF treatment generally refers to applying an electromagnetic field at frequencies from about 1 MHz to about 100 MHz. In one embodiment of the present invention, the RF-energy is within the range of frequencies from about 1 MHz to about 100 MHz, such as from about 5 MHz to 50 MHz, such as from about 10 MHz to about 30MHz. More specific frequencies applied include frequencies of about 24.4 MHz, about 27.12 MHz, about 13.56 MHz and about 40.68MHz.

As said above also other types of electromagnetic energy (EM energy), such as MicroWave (MW) energy, Infra Red (IR) energy and UltraViolet (UV) energy and combinations thereof may be useful in the present invention. Preferred combinations are RF energy and IR energy, RF energy and MW energy, and RF energy, IR energy and MW energy.

The MW energy has a frequency range from about 300 Mhz to about 300 GHz.

The IR energy has a frequency range from about 300 GHz to about 400 THz.

The UV energy has a frequency range from about 400 THz to about 10 PHz.

In one embodiment the EM energy has a frequency such that it is non-ionizing, meaning below about 1000 THz.

The definition of the frequency ranges for RF, MW, IR and UV energies is not standardized and may vary slightly between different text books. The above frequency ranges are among the ranges most commonly used.

The type of EM energy mainly disclosed in the present application is RF energy. What is disclosed on RF energy in the present application is applicable *mutatis mutandis* on the other types of EM energy disclosed in the present application.

In one embodiment, the die and the compaction punch are serving as the electrodes (e.g., one can be the ground electrode) through which RF energy is delivered to the gum-base-comprising powder portion or powder aggregate. In one embodiment, there is direct contact between at least one electrode and the gum-base-comprising powder portion or powder aggregate. In another embodiment, there is no contact between any of the electrodes and the gum shape. In one embodiment, the punches are in direct contact with the surface of the gum-base-comprising powder portion or powder aggregate when the energy is added. In another embodiment, the punches are not in contact (e.g., from about 1 mm to about 1 cm from the surface of the gum-base-comprising powder portion or powder aggregate) during the addition of the energy.

In a preferred embodiment the powder aggregate and at least one of the one or more deposits are concomitantly treated with RF energy.

In one embodiment, the RF energy is delivered once the gum aggregate is shaped. In one embodiment, the energy is delivered continuously starting when the densification begins. In one embodiment, the RF energy is delivered after the gum aggregate has been removed from the die.

The punch and/or the forming die can optionally have electrically insulated side walls and/or can be fully electrically insulated. In such an embodiment, the RF energy can be delivered through insulated electrodes or through electrodes which are not in direct contact with the powder aggregate or separated from the powder aggregate by an air gap. In one embodiment, the die is non-conductive such that it cannot conduct RF energy, in that the energy is directly applied to the powder portion or powder aggregate. In this embodiment, only the punches are conductive. In one embodiment, the die is constructed of plastic, polyethylene, high density polyethylene, polyvinylchloride, polypropylene, high density polypropylene, or Teflon®. In one embodiment, the punches are non-conductive and portions of the die act as two electrodes in order to direct and deliver the RF energy to the powder portion or powder aggregate.

In one embodiment, to help reduce sticking, the chewing gum is cooled within the die. The cooling can be passive cooling (e.g., at room temperature) or active cooling (e.g., coolant recirculation cooling). When coolant recirculation cooling is used, the coolant can optionally circulate through channels inside the punches or punch platen and/or the die or die platen. In one embodiment, the process uses a die platen having multiple die cavities and upper and lower punch platens having multiple upper and lower punched for simultaneous forming of a plurality of chewing gums wherein the platens are actively cooled.

In one embodiment, RF energy is combined with a second source of energy including but not limited to conduction, infrared, induction, or convection heating. In one embodiment the powder portion and/or powder aggregate provides resistance between two non-RF electrodes, and heat is generated as a result of resistance upon the addition of electricity.

### Exterior Deposits

In one embodiment, the chewing gum further comprises at least one deposit (e.g., to add crispiness, enhance taste, provide an alternative or additional source of nicotine and/or buffering agent or protect the gum during storage). Examples of such deposits include, but are not limited to, layers, films, coatings, such as sugar coatings, film coatings, press coatings, compression coatings and melt coatings, beads, tablets, capsules, flakes, granules, pills, pastilles, hard-boiled lozenges, jelly gums and gels and/or combinations thereof, whereby optionally said deposits may be fracture sensitive and may initially comprise powder.

For film and sugar coatings, the coating may be manually placed or sprayed onto the chewing gum product in rotating pans of different shapes or fluidized beds.

Sugar coating is a multistep process and may be divided into the following steps: (i) sealing of the chewing gum product; (ii) subcoating; (iii) smoothing or glossing; (iv) coloring; (v) polishing; and (vi) optionally printing. Sugar coated gums have a smoother profile with less visible edges remaining from the original core. Sub-coating, e.g., either by dusting with powder on the polyol solution or application of dry powder in the polyol solution, may be used. The chewing gum may also be coated by a panning technique, e.g., using a sugar coating pan, or other more sophisticated techniques capable of some degree of automation. The sugar in a sugar coating may be sucrose or other types of sugar, such as sugar alcohols, and/or an artificial sweetener.

Film coating involves the deposition, usually by a spray method, of a thin film of polymer surrounding the chewing gum. The solution may be sprayed on to a rotated, mixed bed. The drying conditions permit the removal of the solvent so as to leave a thin deposition of coating material around each chewing gum.

In one embodiment, the one or more deposits are substantially free of RF-interacting ingredients, in which case application of the RF energy has no significant effect on the deposit itself. In other embodiments, the deposit comprises ingredients that are affected by RF energy, but is devoid of gum base. Such deposits, which initially may comprise powder, may undergo transformation by sintering and/or fusing and/or melting and/or mechanical interlocking, thereby forming a coherent body, which becomes part of the chewing gum.

In another embodiment a deposit such as, but not limited to, directly compressed tablets, beads, capsules, flakes, granules, pills, pastilles, hard-boiled lozenges or jelly gums may be dispensed adjacent to a gum-base-comprising powder portion. Upon RF treatment a unitary chewing gum is obtained.

In one embodiment the nicotine and the buffer are separated from each other by being kept in separate deposits. See further in the below examples.

### Interior Deposits

In one embodiment, a deposit is incorporated into the powder portion or powder aggregate before the RF energy is applied. Useful such deposits include, but are not limited to, beads, tablets, capsules, flakes, granules, pills, pastilles and gels and/or combinations thereof, whereby optionally said deposits may initially comprise powder.

In one embodiment, the nicotine is present in a gel bead, which is liquid filled or semi-solid filled. The gel bead(s) may be added as a part of the powder or the powder blend. In one embodiment, the chewing gum allows for the incorporation of liquid or semisolid filled particles, beads, flakes or other fracture sensitive formats which would have ruptured had they been subjected to the stresses involved in traditional mixing, rolling and scoring or direct compression gum manufacturing.

In one embodiment, the one or more deposits are substantially free of RF-interacting ingredients, in which case application of the RF energy has no significant effect on the deposit itself. In other embodiments, the deposit comprises ingredients that are affected by RF energy but is devoid of gum base. Such deposits, which initially may comprise powder, may undergo transformation by sintering and/or fusing and/or melting and/or mechanical interlocking, thereby forming a coherent body which becomes part of the chewing gum.

In one embodiment the chewing gum comprises at least one exterior deposit and at least one interior deposit.

### Further Embodiments

The present invention may encompass a number of further embodiments, such as
- When a punch is used, said punch may comprise an electrode, which delivers said RF energy to said powder aggregate.
- When a die is used, said die comprises an electrode, which delivers said RF energy to said powder aggregate.
- When a die and punches are used said gum-base comprising powder is densified using an upper punch and a lower punch, and at least one of
- said upper punch or lower punch comprises an electrode, which delivers said RF energy to said powder aggregate.
- At least one of forming, making and adhering of one or more of said one or more deposit(s) takes place concomitantly with the processing of the gum base-comprising powder.
- At least one of forming, making and adhering of one or more of said one or more deposit(s) take(s) place separately from the processing of the gum base-comprising powder.
- At least one of said one or more deposit(s) is made using application of RF energy.
- At least one of said one or more deposit(s) is made using any of compression, compaction, slugging, coating, molding, extrusion and/or granulation.
- The adhering of said one or more deposit(s) is achieved by application of RF energy.
- At least one of said one or more deposits provides a crispy and/or crunchy mouth-feel to a person chewing said chewing gum.
- Incompatible ingredients of the chewing gum are separated from each other by being located in separate parts of the chewing gum.
- One or more deposits are located at least partly at the peripheral part of the chewing gum.
- One or more deposits are located at least partly within the chewing gum.
- The gum-base comprising powder is a blend of powders with different properties.
- The one or more deposits are different between themselves.
- Upon having been produced the chewing gum is further treated with NI energy and/or thermal and/or mechanical energy.

### Use of Chewing Gum

Disclosed but not claimed is a method of treating tobacco
dependence and/or providing satisfaction equivalent to the satisfaction experienced from use of tobacco, such a smoking or use of smoke-less tobacco.

In this disclosure, a unit dose is typically accompanied by dosing directions, which instruct the patient to take an appropriate amount of the nicotine that may be a multiple of the unit dose depending on, e.g. how strong the patient's tobacco dependence is.

### Examples

Specific embodiments of the present invention are illustrated by way of the following examples. This invention is not confined to the specific limitations set forth in these examples. The below examples were carried out in laboratory scale batch size as well as was used desk top RF treatment equipment using typically 4 kW at 27.1 MHz. When using production scale RF equipment the RF treatment time will be accordingly adjusted including adaption of RF power and RF treatment time.

### Example 1: Preparation of Placebo Chewing Gum

The powder blend of Table 1 is prepared as follows. The colorant, flavor, acesulfame K, and sucralose are manually passed through a 50 mesh screen. The above mixture and remaining materials are added to a plastic bottle, mixed end-over end for approximately three minutes, and then discharged. The powder blend is then individually dispensed into a simulated tablet-like medicament die utilizing 1000 mg of the blend per die. The die is constructed of a non-conductive plastic and the punches act as electrodes within an RF unit. The powder portions are then treated with RF energy for 15 seconds to transform the powder portion into a chewing gum. The chewing gum is then ejected from the die.

**Table 1**

| Material | g/batch | mg/gum | weight % |
|---|---|---|---|
| HiG PWD-03 Gum Base¹ | 97.01 | 970.05 | 97.01 |
| Blue Lake Colorant | 0.02 | 0.20 | 0.02 |
| Vanilla-Mint Flavor | 1.00 | 10.00 | 1.00 |
| Peppermint Flavor | 0.50 | 5.00 | 0.50 |
| Sodium Bicarbonate anhydrous | 0.50 | 5.00 | 0.50 |
| Acesulfame K | 0.20 | 2.00 | 0.20 |
| Sucralose Powder | 0.40 | 4.00 | 0.40 |
| Amorphous Silica | 0.38 | 3.75 | 0.38 |
| TOTAL | 100.0 | 1000.00 | 100.0 |

| | | | |
|---|---|---|---|
| 1: Commercially available from the Cafosa Corporation in Barcelona, Spain; comprises gum base, isomalt, sorbitol and an anticaking agent. | | | |

### Example 2: Preparation of Chewing Gum Containing Nicotine Bitartrate Dihydrate

The powder blend of Table 2 is prepared as follows. The colorant, flavor, acesulfame K, and sucralose are manually passed through a 50 mesh screen. The above mixture and remaining materials including the nicotine bitartrate dihydrate are added to a plastic bottle, mixed end-over end for approximately three minutes, and then discharged. The powder blend is then individually dispensed into a simulated tablet-like medicament die utilizing 1000 mg of the blend per die. The die is made by a non-conductive plastic and the punches act as electrodes within an RF unit. The powder portions are then treated with RF energy for 15 seconds to transform the powder portion into a chewing gum. The chewing gum is then ejected from the die.

**Table 2**

| Material | g/batch | mg/gum | weight % |
|---|---|---|---|
| HiG PWD-03 Gum Base¹ | 96.390 | 963.90 | 96.390 |
| Nicotine Bitartrate Dihydrate (32.55% Nicotine)* | 0.615 | 6.15* | 0.615 |
| Blue Lake Colorant | 0.020 | 0.20 | 0.020 |
| Vanilla-Mint Flavor | 1.000 | 10.00 | 1.000 |
| Peppermint Flavor² | 0.500 | 5.00 | 0.500 |
| Sodium Carbonate anhydrous | 0.500 | 5.00 | 0.500 |
| Acesulfame K | 0.200 | 2.00 | 0.200 |
| Sucralose Powder | 0.400 | 4.00 | 0.400 |
| Amorphous Silica | 0.375 | 3.75 | 0.375 |
| TOTAL | 100.00 | 1000.00 | 100.000 |

| | | | |
|---|---|---|---|
| *Equivalent to 2.0 mg of Nicotine 1: Commercially available from the Cafosa Corporation in Barcelona, Spain; comprises gum base, isomalt, sorbitol and an anticaking agent. 2: Commercially available from Virginia Dare in Brooklyn, NY | | | |

### Example 3: Preparation of Chewing Gum Containing Nicotine Resin Complex

The powder blend of Table 3 is prepared as follows. The colorant, flavor, acesulfame K, and sucralose are manually passed through a 50 mesh screen. The above mixture and remaining materials including the nicotine resin complex and the Trometamol are added to a plastic bottle, mixed end-over end for approximately three minutes, and then discharged. The powder blend is then individually dosed into a simulated tablet-like medicament die utilizing 1000 mg of the blend per die. The die is constructed of a non-conductive plastic and the punches act as electrodes within an RF unit. The gum shapes are then heated and activated utilizing RF energy for 15 seconds to sinter the granulation into a unified chewing gum product. The chewing gum product is then ejected from the die.

**Table 3**

| Material | g/batch | mg/gum | weight % |
|---|---|---|---|
| HiG PWD-03 Gum Base¹ | 92.72 | 927.2 | 92.72 |
| Nicotine Resin Complex (20% Nicotine) | 1.00 | 10.0 | 1.00 |
| Trometamol | 3.30 | 33.0 | 3.30 |
| Vanilla-Mint Flavor | 1.00 | 10.0 | 1.00 |
| Peppermint Flavor² | 0.50 | 5.0 | 0.50 |
| Sodium Bicarbonate anhydrous | 0.50 | 5.0 | 0.50 |
| Acesulfame K (sweetener) | 0.20 | 2.0 | 0.20 |
| Sucralose Powder (sweetener) | 0.40 | 4.0 | 0.40 |
| Amorphous Silica | 0.38 | 3.8 | 0.38 |
| TOTAL | 100.00 | 1000.0 | 100.00 |

| | | | |
|---|---|---|---|
| *Equivalent to a 2.0 mg Dose of Nicotine. 1: Commercially available from the Cafosa Corporation in Barcelona, Spain; comprises gum base, isomalt, sorbitol and an anticaking agent. 2: Commercially available from Virginia Dare in Brooklyn, NY | | | |

### Example 4. Preparation of Chewing Gum Containing Nicotine Resin Complex

The powder blend of Table 4 is prepared as follows. The colorant, flavor, acesulfame K, and sucralose are manually passed through a 50 mesh screen. The above mixture and remaining materials including the nicotine resin complex are added to a planetary mixer type Kitchen Aid and mixed for approximately five minutes, then magnesium stearate is added and mixed for a period of additionally 2.5 min and the material is then discharged. The powder blend is then individually dosed into a simulated tablet-like medicament die utilizing 1000 mg of the blend per die. The die is constructed of a non-conductive plastic and the punches act as electrodes within an RF unit. The gum shapes are then heated by RF energy for 15 seconds to sinter the granulation into a unified chewing gum product. The chewing gum product is then ejected from the die.

**Table 4**

| Material | g/batch | mg/gum | weight % |
|---|---|---|---|
| HiG PWD-03 Gum Base¹ | 95.00 | 950.00 | 95.00 |
| Nicotine Resin Complex (20% Nicotine) | 1.00 | 10.00* | 1.00 |
| Blue Lake Colorant | 0.02 | 0.20 | 0.02 |
| Mint Flavor | 1.00 | 10.00 | 1.00 |
| Peppermint Flavor² | 0.50 | 5.00 | 0.50 |
| Sodium Bicarbonate anhydrous | 0.50 | 5.00 | 0.50 |
| Acesulfame K (sweetener) | 0.20 | 2.00 | 0.20 |
| Sucralose Powder (sweetener) | 0.40 | 4.00 | 0.40 |
| Silicon dioxide | 0.38 | 3.80 | 0.38 |
| Magnesium stearate | 1.00 | 10.00 | 1.00 |
| TOTAL | 100.00 | 1000.00 | 100.00 |

| | | | |
|---|---|---|---|
| *Equivalent to a 2.0 mg Dose of Nicotine 1: Commercially available from the Cafosa Corporation in Barcelona, Spain; comprises gum base, isomalt, sorbitol and an anticaking agent. 2: Commercially available from Givaudan | | | |

### Example 5. Preparation of Chewing Gum Comprising Nicotine Resin Complex

All materials are sieved using a 1 mm sieve. HiG PWD-03 Gum Base, powder flavor and sweetener is added to a planetary mixer type Kitchen Aid and mixed for 5 minutes. Liquid mint flavor is added by spraying in intervals during mixing. Silicon dioxide is added immediately after adding the liquid flavor and mixing is continued for an additional 1 minute. The last step is the addition of Magnesium stearate and mixing for 2.5 minutes. The powder blend is then individually dosed into a simulated tablet-like medicament die utilizing 1000 mg of the blend per die. The die is constructed of a non-conductive plastic and the punches act as electrodes within an RF unit. The gum shapes are then treated with RF energy for 15 seconds to sinter the granulation into a unified chewing gum product. The chewing gum product is then ejected from the die.

**Table 5**

| Material | g/batch | mg/gum | weight % |
|---|---|---|---|
| HiG PWD-03 Gum Base¹ | 94.40 | 944.00 | 94.40 |
| Nicotine Resin Complex (20% Nicotine) | 2.00 | 20.00* | 2.00 |
| Peppermint Liquid Flavor² | 0.50 | 5.00 | 0.50 |
| Peppermint Powder Flavor² | 0.50 | 5.00 | 0.50 |
| Acesulfame K (sweetener) | 0.20 | 2.00 | 0.20 |
| Sucralose (sweetener) | 0.40 | 4.00 | 0.40 |
| Amorphous Silica | 1.00 | 10.00 | 1.00 |
| Magnesium stearate | 1.00 | 10.00 | 1.00 |
| TOTAL | 100.00 | 1000.00 | 100.00 |

| | | | |
|---|---|---|---|
| *Equivalent to a 4.0 mg Dose of Nicotine 1: Commercially available from the Cafosa Corporation in Barcelona, Spain; comprises gum base, isomalt, sorbitol and an anticaking agent. 2: Commercially available from Symrise | | | |

### Example 6: Preparation of Bi-Layer Chewing Gum

The powder blend of Table 6a is prepared as follows ("Gum Powder Blend"). The colorant, flavor, acesulfame K, and sucralose are manually passed through a 50 mesh screen. The above mixture and remaining materials including the nicotine resin complex and the sodium bicarbonate and sodium carbonate are added to a plastic bottle, mixed end-over-end for approximately three minutes, and then discharged.

**Table 6a**

| Material | g/batch | mg/gum | weight % |
|---|---|---|---|
| HiG PWD-03 Gum Base¹ | 97.61 | 976.10 | 97.61 |
| Nicotine Resin Complex (20% Nicotine) | 1.00 | 10.00* | 1.00 |
| Sodium Bicarbonate USP | 0.25 | 2.50 | 0.25 |
| Sodium Carbonate, Anhydrous | 0.50 | 5.00 | 0.50 |
| D&C Red Lake #7 Colorant | 0.04 | 0.40 | 0.04 |
| Acesulfame K (sweetener) | 0.20 | 2.00 | 0.20 |
| Sucralose Powder (sweetener) | 0.40 | 4.00 | 0.40 |
| TOTAL | 100.0 | 1000.00 | 100.0 |

| | | | |
|---|---|---|---|
| *Equivalent to a 2.0 mg Dose of Nicotine 1: Commercially available from the Cafosa Corporation in Barcelona, Spain; comprises gum base, isomalt, sorbitol and an anticaking agent. | | | |

The powder blend of Table 6b ("Isomalt Powder Blend") is prepared by adding the Galen IQ, the cinnamon, the sucralose and the sodium stearyl fumarate into a plastic bottle and mixing end-over-end for approximately 3 minutes and then discharged.

**Table 6b**

| Material | g/batch | mg/gum | weight % |
|---|---|---|---|
| Galen IQ 720 Directly Compressible Isomalt¹ | 89.30 | 267.90 | 89.30 |
| Spray Dried Cinnamon flavor | 10.00 | 30.00* | 10.00 |
| Sucralose | 0.20 | 0.60 | 0.20 |
| Sodium Stearyl Fumarate | 0.50 | 1.50 | 0.50 |
| TOTAL | 100.0 | 300.00 | 100.0 |

| | | | |
|---|---|---|---|
| 1: Commercially available from the BENEO-Palatinit GmbH Corporation in Manheim, Germany | | | |

300 mg of the Isomalt Powder Blend is added to the die and compressed at approximately 5 kP. Then, 1000 mg of the Gum Powder Blend is then added to the compacted isomalt layer within the die, and treated with RF energy for 15 seconds to sinter the isomalt layer and the gum powder blend into a unified bilayer dosage form. The bilayer chewing gum is then ejected from the die.

### Example 7. Preparation of Chewing Gum Containing Nicotine Resin Complex with gum base content of 50%.

The powder blend of Table 7 is prepared as follows. Isomalt, Sodium carbonate anhydrous, Sodium hydrogen carbonate, Acesulfame Potassium, Sucralose, flavour in powder form and Magnesium oxide are sieved and loaded to a powder mixer together with the Nicotine Resinate. The raw materials are then mixed together to form a powder premix.

**Table 7**

| | 0 mg Unit Formula (mg) | 0.5 mg Unit Formula (mg) | 1 mg Unit Formula (mg) | 2 mg Unit Formula (mg) | 3 mg Unit Formula (mg) | 4 mg Unit Formula (mg) |
|---|---|---|---|---|---|---|
| Nicotine Resin Complex 20% | 0 | 2.5 | 5 | 10 | 15 | 20 |
| Flavor Powder Form² | 30 | 30 | 30 | 30 | 30 | 30 |
| Sodium Hydrogen Carbonate | 20 | 20 | 15 | 10 | 5 | - |
| Sodium Carbonate | 10 | 10 | 15 | 20 | 25 | 30 |
| Magnesium Stearate | 15 | 15 | 15 | 15 | 15 | 15 |
| Acesulfame K | 2 | 2 | 2 | 2 | 2 | 2 |
| Amorphous Silica | 5 | 5 | 5 | 5 | 5 | 5 |
| Sucralose | 1 | 1 | 1 | 1 | 1 | 1 |

| | | | | | | |
|---|---|---|---|---|---|---|
| 1: Commercially available from the Cafosa Corporation in Barcelona, Spain. 2: Commercially available from Givaudan. | | | | | | |

At low temperature the chewing gum base is milled together with amorphous silica and passed through a 1,0 mm screen. The milled gum base and amorphous silica are then added to the powder premix and mixed to form a homogenous distribution of the ingredients, finally the magnesium stearate is added and mixed for a few minutes. The powder blend is then individually dosed into a simulated tablet-like medicament die utilizing 1000 mg of the blend per die. The die is constructed of a non-conductive plastic and the punches act as electrodes within an RF unit. The gum shapes are then heated utilizing RF energy for 30 seconds to sinter the granulation into a unified chewing gum product. The chewing gum product is then ejected from the die. Also other percentages of gum base content are possible, e g from about 10 % to about 80 %.

### Example 8. Preparation of Chewing Gum Containing Nicotine Resin Complex with gum base content >20%.

The powder blend of Table 8 is prepared as follows. The Chewing Gum Base, Sodium carbonate anhydrous, Sodium hydrogen carbonate, Acesulfame Potassium, Sucralose and Magnesium oxide are sieved and loaded to a powder mixer together with the encapsulated flavours and Nicotine Resinate. The raw materials are then mixed together to form a homogenous distribution of the ingredients, finally the magnesium stearate is added and mixed for a few minutes.

**Table 8**

| **Active ingredient** | 0 mg | 0.5 mg | 1 mg | 2 mg | 3 mg | 4 mg |
|---|---|---|---|---|---|---|
| | Unit formula (mg) | Unit formula (mg) | Unit formula (mg) | Unit formula (mg) | Unit formula (mg) | Unit formula (mg) |
| Nicotine resin complex 20% | 0 | 2,5 | 5 | 10 | 15 | 20 |

| **Other ingredients** | | | | | | |
|---|---|---|---|---|---|---|
| Chewing gum base for compression (HiG PWD-03)¹ | 905 | 902,5 | 900 | 895 | 890 | 885 |
| Encapsulated flavour CapLock Peppermint² | 20 | 20 | 20 | 20 | 20 | 20 |
| Flavour in powder form Peppermint2² | 20 | 20 | 20 | 20 | 20 | 20 |
| Sodium hydrogen | 20 | 20 | 15 | 10 | 5 | - |
| carbonate | | | | | | |
| Sodium carbonate | 10 | 10 | 15 | 20 | 25 | 30 |
| Magnesium stearate | 15 | 15 | 15 | 15 | 15 | 15 |
| Magnesium oxide | 5 | 5 | 5 | 5 | 5 | 5 |
| Acesulfame Potassium | 2 | 2 | 2 | 2 | 2 | 2 |
| Sucralose | 3 | 3 | 3 | 3 | 3 | 3 |
| | 1000 | 1000 | 1000 | 1000 | 1000 | 1000 |

| | | | | | | |
|---|---|---|---|---|---|---|
| 1: Commercially available from the Cafosa Corporation in Barcelona, Spain. 2: Commercially available from IFF. | | | | | | |

The powder blend is then individually dosed into a simulated tablet-like medicament die utilizing 1000 mg of the blend per die. The die is constructed of a non-conductive plastic and the punches act as electrodes within an RF unit. The gum shapes are then treated utilizing RF energy for 15 seconds to sinter the granulation into a unified chewing gum product. The chewing gum product is then ejected from the die.

The amount of buffers may be adjusted to achieve desired nicotine absorption kinetics.

### Example 9: Preparation of Bi-Layer Chewing Gum Cinnamon with Polydextrose layer

The powder blend of Table 9a is prepared as follows ("Gum Powder Blend"). The colorant, flavor, acesulfame K, and sucralose are manually passed through a 50 mesh screen. The above mixture and remaining materials including the nicotine resin complex and the sodium bicarbonate and sodium carbonate are added to a plastic bottle, mixed end-over-end for approximately three minutes, and then discharged.

**Table 9a**

| Material | g/batch | mg/gum | weight % |
|---|---|---|---|
| HiG PWD-03 Gum Base¹ | 97.51 | 975.10 | 97.51 |
| Nicotine Resin Complex (20% Nicotine) | 1.10 | 11.00* | 1.10 |
| Sodium Bicarbonate USP | 0.25 | 2.50 | 0.25 |
| Sodium Carbonate, Anhydrous | 0.50 | 5.00 | 0.50 |
| D&C Red Lake #7 Colorant | 0.04 | 0.40 | 0.04 |
| Acesulfame K (sweetener) | 0.20 | 2.00 | 0.20 |
| Sucralose Powder (sweetener) | 0.40 | 4.00 | 0.40 |
| TOTAL | 100.00 | 1000.00 | 100.00 |

| | | | |
|---|---|---|---|
| *Equivalent to a 2.2 mg Dose of Nicotine 1: Commercially available from the Cafosa Corporation in Barcelona, Spain; comprises gum base, isomalt, sorbitol and an anticaking agent. | | | |

The polydextrose powder blend of Table 9b is prepared by adding the polydextrose, the cinnamon, the sucralose and the sodium stearyl fumarate into a plastic bottle and mixing end-over-end for approximately 3 minutes and then discharged.

**Table 9b**

| Material | g/batch | mg/gum | weight % |
|---|---|---|---|
| Polydextrose^{1,2} | 89.30 | 267.90 | 89.30 |
| Spray Dried Cinnamon flavor | 10.00 | 30.00 | 10.00 |
| Sucralose | 0.20 | 0.60 | 0.20 |
| Sodium Stearyl Fumarate | 0.50 | 1.50 | 0.50 |
| TOTAL | 100.00 | 300.00 | 100.00 |

| | | | |
|---|---|---|---|
| 1: Commercially available from Danisco, Denmark 2: Polydextrose may be exchanged for hydrogenated starch hydrosylate or cornstarch. | | | |

300 mg of the Polydextrose is added to the die and densified at approximately 5 kP. Then, 1000 mg of the Gum Powder Blend is added to the polydextrose layer within the die, and treated utilizing RF energy for 15 seconds to sinter the isomalt layer and the gum blend into a unified bilayer dosage form. The bilayer chewing gum is then ejected from the die.

### Example 10: Preparation of Bi-Layer Mint Chewing Gum with Polydextrose layer

The powder blend of Table 10a is prepared as follows ("Gum Powder Blend"). The colorant, flavor, acesulfame K, and sucralose are manually passed through a 50 mesh screen. The above mixture and remaining materials including the nicotine resin complex and the sodium bicarbonate and sodium carbonate are added to a plastic bottle, mixed end-over-end for approximately three minutes, and then discharged.

**Table 10a**

| Material | g/batch | mg/gum | weight % |
|---|---|---|---|
| HiG PWD-03 Gum Base¹ | 97.51 | 975.10 | 97.51 |
| Nicotine Resin Complex (20% Nicotine) | 1.10 | 11.00* | 1.10 |
| Sodium Bicarbonate USP | 0.25 | 2.50 | 0.25 |
| Sodium Carbonate, Anhydrous | 0.50 | 5.00 | 0.50 |
| Mint | 0.04 | 0.40 | 0.04 |
| Acesulfame K (sweetener) | 0.20 | 2.00 | 0.20 |
| Sucralose Powder (sweetener) | 0.40 | 4.00 | 0.40 |
| TOTAL | 100.00 | 1000.00 | 100.00 |

| | | | |
|---|---|---|---|
| *Equivalent to a 2.2 mg Dose of Nicotine 1: Commercially available from the Cafosa Corporation in Barcelona, Spain; comprises gum base, isomalt, sorbitol and an anticaking agent. | | | |

The polydextrose powder blend of Table 10b is prepared by adding the polydextrose, the mint flavor, the sucralose and the sodium stearyl fumarate into a plastic bottle and mixing end-over-end for approximately 3 minutes and then discharged.

**Table 10b**

| Material | g/batch | mg/gum | weight % |
|---|---|---|---|
| Polydextrose^{1,2} | 89.30 | 267.90 | 89.30 |
| Spray Dried Mint Flavor | 10.00 | 30.00 | 10.00 |
| Sucralose | 0.20 | 0.60 | 0.20 |
| Sodium Stearyl Fumarate | 0.50 | 1.50 | 0.50 |
| TOTAL | 100.0 | 300.00 | 100.0 |

| | | | |
|---|---|---|---|
| 1: Commercially available from Danisco, Denmark 2: Polydextrose may be exchanged for Hydrogenated starch hydrosylate or cornstarch. | | | |

300 mg of the Polydextrose is added to the die and densified at approximately 5 kP. Then, 1000 mg of the Gum Powder Blend is added to the polydextrose layer within the die, and treated utilizing RF-energy for 15 seconds to sinter the polydextrose layer and the gum blend into a unified bilayer dosage form. The bilayer chewing gum product is then ejected from the die.

### Example 11. Preparation of Bi-Layer Chewing Gum Product with Crispy Polyol Layer comprising Nicotine Resin Complex in Both Layers

As Example 6, but the polyol layer containing isomalt also contains 1 mg nicotine resinate and the amount of isomalt is reduced with 5 mg and is compressed using 30 kN (15 mm round concave punch) in a separate compression step whereafter the chewing gum powder blend is added, a shape forming, but low, compaction pressure is added and RF-energy is applied for 15 seconds.

### Example 12. Preparation of a Crunchy Chewing Gum Containing Nicotine Resin Complex

All materials are sieved. Thereafter all the materials, except magnesium stearate, are added to a planetary mixer type Kitchen Aid and mixed for 5 minutes. Last, Magnesium Stearate is added and mixed for 2.5 minutes.

The powder blend is then individually dosed into a simulated tablet-like medicament die utilizing 1000 mg of the blend per die. The die is constructed of a non-conductive plastic and the punches act as electrodes within an RF unit. The gum shapes are then treated utilizing RF energy for 15 seconds to sinter the granulation into a unified chewing gum product. The chewing gum product is then ejected from the die.

**Table 12**

| Material | g/batch | mg/gum | weight % |
|---|---|---|---|
| HiG PWD-03 Gum Base¹ | 84.00 | 840.00 | 84.00 |
| Nicotine Resin Complex (20% Nicotine) | 2.00 | 20.00* | 2.00 |
| Polydextrose granulated | 11.30 | 113.00 | 11.30 |
| Peppermint Liquid Flavor² | 0.50 | 5.00 | 0.50 |
| Peppermint Powder Flavor² | 0.50 | 5.00 | 0.50 |
| Acesulfame K (sweetener) | 0.20 | 2.00 | 0.20 |
| Sucralose (sweetener) | 0.40 | 4.00 | 0.40 |
| Amorphous Silica | 1.00 | 10.00 | 1.00 |
| Magnesium stearate | 1.00 | 10.00 | 1.00 |
| TOTAL | 100.00 | 1000.00 | 100.00 |

| | | | |
|---|---|---|---|
| *Equivalent to a 4.0 mg Dose of Nicotine 1: Commercially available from the Cafosa Corporation in Barcelona, Spain; comprises gum base, isomalt, sorbitol and an anticaking agent. 2: Commercially available from A.M. Todd | | | |

### Example 13: Preparation of Nicotine Chewing Gum Comprising Encapsulated Flavor System

An encapsulated flavor is utilized to ensure flavor stability over the entire shelf life of the product. The powder blend of Table 13 is prepared as follows. The flavor, acesulfame K, and sucralose are manually passed through a 50 mesh screen. The above mixture and remaining materials including the nicotine resin complex are added to a Turbula mixer, mixed end-over end for approximately eight minutes, and then discharged. The powder blend is then individually dosed into a simulated tablet-like medicament die utilizing 1000 mg of the blend per die. The die is constructed of a non-conductive plastic and the punches act as electrodes within an RF unit. The gum shapes are then treated with RF energy for 15 seconds to sinter the powder into a unified chewing gum product. The chewing gum product is then ejected from the die.

**Table 13**

| Material | g/batch | mg/gum | weight % |
|---|---|---|---|
| HiG PWD-03 Gum Base | 94.520 | 945.20 | 94.52 |
| Nicotine Resin Complex (20% Nicotine) | 1.000 | 10.00* | 1.00 |
| Encapsulated Fruit Flavor¹ | 2.000 | 20.00 | 2.00 |
| Sodium Carbonate Anhydrous | 1.000 | 10.00 | 1.00 |
| Sodium Bicarbonate anhydrous | 0.500 | 5.00 | 0.50 |
| Acesulfame K (sweetener) | 0.200 | 2.00 | 0.20 |
| Sucralose Powder (sweetener) | 0.400 | 4.00 | 0.40 |
| Amorphous Silica | 0.380 | 3.80 | 0.38 |
| TOTAL | 100.000 | 1000.00 | 100.00 |

| | | | |
|---|---|---|---|
| *Equivalent to a 2.0 mg Dose of Nicotine 1: Commercially available from Givaudan | | | |

### Example 14: Preparation of Nicotine Chewing Gum Comprising Three Layers For Separation of Ingredients

Three separate powder blend layers, where one layer is a pre-compacted layer comprising polyol and the other two layers comprise gum base, are sintered together to form a coherent chewing gum product. This procedure allows for the separation of ingredients with compatibility issues. The three powder blends of Table 14 are prepared as follows. Powder blends 1, 2 and 3 are added to separate plastic bottles and mixed end-over end for approximately three minutes. Blend 1, comprising polyol, is then compressed using 30 kN (15 mm round punch) in a separate step whereafter the two gum base-comprising powder blends are consecutively added, forming a three-layered matrix utilizing a total amount of 1300 mg material per die. Finally, a shape forming but low compaction pressure is added and RF-energy is applied for 15 seconds to sinter the blends into a unified chewing gum product. The chewing gum product is then ejected from the die.

**Table 14**

| Material | g/batch | mg/gum | weight % |
|---|---|---|---|
| Blend 1: Polyol layer | | | |
| Galen IQ 720 Directly Compressible Isomalt | 29.490 | 294.90 | 22.68 |
| Cinnamon Flavor | 0.300 | 3.00 | 0.23 |
| Sucralose Powder (sweetener) | 0.060 | 0.60 | 0.05 |
| Magnesium Stearate | 0.150 | 1.50 | 1.15 |
| Total Blend 1 | 30.000 | 300.00 | 23.08 |

| Blend 2: Nicotine-comprising gum layer | | | |
|---|---|---|---|
| HiG PWD-03 Gum Base | 47.000 | 470.00 | 36.15 |
| Nicotine Resin Complex (20% Nicotine) | 1.000 | 10.00* | 0.77 |
| Sucralose Powder (sweetener) | 0.300 | 3.00 | 0.23 |
| Sodium Carbonate Anhydrous | 0.500 | 5.00 | 0.38 |
| Sodium Bicarbonate anhydrous | 1.000 | 10.00 | 0.77 |
| Amorphous Silica | 0.200 | 2.00 | 0.15 |
| Total Blend 2 | 50.000 | 500.00 | 38.46 |

| Blend 3: Flavor-comprising gum layer | | | |
|---|---|---|---|
| HiG PWD-03 Gum Base | 48.400 | 484.00 | 37.23 |
| Cinnamon Flavor | 1.200 | 12.00 | 0.92 |
| Acesulfame K (sweetener) | 0.200 | 2.00 | 0.15 |
| Amorphous Silica | 0.200 | 2.00 | 0.15 |
| Total Blend 3 | 50.000 | 500.00 | 38.46 |
| TOTAL | 130.00 | 1300.00 | 100.00 |

| | | | |
|---|---|---|---|
| *Equivalent to a 2.0 mg Dose of Nicotine | | | |

### Example 15: Preparation of Nicotine Chewing Gum Comprising Two Layers, One of Which is Compacted Using Common Tablet Compression Technique

Two separate powder blend layers, where one layer is a pre-compacted layer comprising polyol and the other layer comprise gum base, are sintered together to form a unified chewing gum product. The two powder blends of Table 15 are prepared as follows. Powder blend 1 is added to a plastic bottle and mixed end-over end for approximately three minutes. Blend 1, comprising polyol, is then compressed using 30 kN (15 mm round punch) in a separate step.

The powder blend 2 is prepared as follows. Isomalt, Sodium carbonate anhydrous, Sodium hydrogen carbonate, Acesulfame Potassium, Sucralose , flavour in powder form and Magnesium oxide are sieved and loaded to a powder mixer together with the Nicotine Resinate. The raw materials are then mixed together to form a powder blend. At low temperature the chewing gum base is milled together with amorphous silica and passed through a 1,0 mm screen. The milled gum base and amorphous silica are then added to the powder premix and mixed to form a homogenous distribution of the ingredients, finally the magnesium stearate is added and mixed for a few minutes.

The compressed tablets created from blend 1 are placed into simulated tablet-like medicament die and the blend 2, gum base-comprising powder blend is consecutively added, forming a two-layered matrix utilizing a total amount of 1300 mg material per die. Finally, a shape forming but low compaction pressure is added and RF-energy is applied for 15 seconds to sinter the blend and pre-compacted layer into a unified chewing gum product. The chewing gum product is then ejected from the die. Also other percentages of gum base content are possible, e g from about 10 % to about 80 %. The amount of buffers may be adjusted to achieve desired nicotine absorption kinetics.

**Table 15**

| | 2 mg | 4 mg |
|---|---|---|
| | Unit formula | Unit formula |
| | (mg) | (mg) |
| **Blend 1: Polyol layer** | | |
| Galen IQ 720 Directly Compressible Isomalt | 294.9 | 294.9 |
| Mint Flavor in powder form | 3 | 3 |
| Sucralose Powder (sweetener) | 0.6 | 0.6 |
| Magnesium Stearate | 1.5 | 1.5 |
| Total Blend 1 | 300 | 300 |
| | | |

| **Blend 2: Gum layer** | | |
|---|---|---|
| Nicotine resin complex 20 % | 10 | 20 |
| Chewing gum base¹ | 500 | 500 |
| Isomalt | 352 | 342 |
| Sorbitol | 50 | 50 |
| Flavour in powder form² | 30 | 30 |
| Sodium hydrogen carbonate | 10 | - |
| Sodium carbonate | 20 | 30 |
| Magnesium stearate | 15 | 15 |
| Magnesium oxide | 5 | 5 |
| Acesulfame K | 2 | 2 |
| Amorphous silica | 5 | 5 |
| Sucralose | 1 | 1 |
| Total Blend 2 | 1000 | 1000 |
| Total Blend 1 and 2 | 1300 | 1300 |

| | | |
|---|---|---|
| 1: Commercially available from the Cafosa Corporation in Barcelona, Spain. 2: Commercially available from Givaudan | | |

### Example 16: Preparation of Bi-layer Nicotine Chewing Gum Comprising Effervescent Agents

This preparation utilizes the increased excretion of saliva at mastication of chewing gum to trigger a carbon dioxide releasing reaction of effervescent agents in one of the layers. A fizzy sensation in the mouth is thus created when using the chewing gum. Two separate powder blend layers, where one layer is a pre-compacted layer comprising effervescent agents and the other layer comprise gum base, are sintered together to form a coherent chewing gum product. The two powder blends of Table 16 are prepared as follows. Powder blends 1 and 2 are added to separate plastic bottles and mixed end-over end for approximately three minutes. Blend 1, comprising effervescent agents, is then compressed using 30 kN (15 mm round punch) in a separate step where after the gum base-comprising powder blend is added, forming a bi-layer matrix utilizing a total amount of 1300 mg material per die. Finally, a shape forming but low compaction pressure is added and RF-energy is applied for 45 seconds to sinter the blends into a unified chewing gum product. The chewing gum product is then ejected from the die.

**Table 16**

| Material | g/batch | mg/gum | weight % |
|---|---|---|---|
| Blend 1: Effervescent layer | | | |
| Galen IQ 720 Directly Compressible | 15.290 | 152.90 | 11.76 |
| Isomalt | | | |
| Sodium Bicarbonate anhydrous | 10.000 | 100.00 | 7.69 |
| Citric Acid Anhydrous | 4.000 | 40.00 | 3.08 |
| Peppermint Flavor | 0.500 | 5.00 | 0.38 |
| Sucralose Powder (sweetener) | 0.060 | 0.60 | 0.05 |
| Magnesium Stearate | 0.150 | 1.50 | 0.12 |
| Total Blend 1 | 30.000 | 300.00 | 23.08 |

| Blend 2: Nicotine-comprising gum layer | | | |
|---|---|---|---|
| HiG PWD-03 Gum Base | 95.020 | 950.20 | 73.09 |
| Nicotine Resin Complex (20% Nicotine) | 2.000 | 20.00* | 1.54 |
| Peppermint Flavor | 1.500 | 15.00 | 1.15 |
| Sodium Bicarbonate anhydrous | 0.500 | 5.00 | 0.39 |
| Acesulfame K (sweetener) | 0.200 | 2.00 | 0.15 |
| Sucralose Powder (sweetener) | 0.400 | 4.00 | 0.31 |
| Amorphous Silica | 0.380 | 3.80 | 0.29 |
| Total Blend 2 | 100.000 | 1000.00 | 76.92 |
| TOTAL | 130.00 | 1300.00 | 100.00 |

| | | | |
|---|---|---|---|
| *Equivalent to a 4.0 mg Dose of Nicotine | | | |

Also other combinations with nicotine are within the scope of the invention.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. A process for making a nicotine-comprising chewing gum, said process comprising the steps of
i. dispensing a powder portion from a gum-base-comprising powder,
ii. optionally shaping said powder portion into a powder aggregate,
iii. and applying sufficient electromagnetic energy (EM energy) to said powder portion or said powder aggregate to transform said powder portion or said powder aggregate into said nicotine-comprising chewing gum,
whereby said EM energy is Radio Frequency (RF) energy, MicroWave (MW) energy, InfraRed (IR) energy or UltraViolet (UV) energy or combinations thereof, preferably Radio Frequency (RF) energy, the combination of RF energy and IR energy, the combination of RF energy and MW energy, and the combination of RF energy, IR energy and MW energy.

2. The process of claim 1, wherein said chewing gum further comprises one or more deposits, such deposits preferably, but not exclusively, being chosen among layers, films, coatings, such as sugar coatings, film coatings, press coatings, compression coatings and melt coatings, beads, tablets, capsules, flakes, granules, pills, pastilles, hard-boiled lozenges, jelly gums and gels and/or combinations thereof, whereby optionally said deposits may initially comprise powder, whereby one or more deposits may be placed on the outside of the chewing gum (exterior deposit) or one or more deposits may be placed within the chewing gum (interior deposit) or at least one deposit is an exterior deposit and at least one deposit is an interior deposit.

3. The process of claim 1, wherein said powder portion is dispensed into a mold, die, other cavity or other shape-forming means.

4. The process of any preceding claim, wherein said optional shaping of said powder portion into a powder aggregate comprises densification, such as one or more of tamping, compression, compacting, de-aeration, vacuum-forming, slugging, granulation and vibration.

5. The process of any of claims 1-4, wherein the transformation of the powder portion and/or the powder aggregate into a chewing gum is obtained by sintering and/or fusing and/or melting and/or mechanical interlocking.

6. The process of any of claims 1 - 5, wherein said RF energy is applied to said powder portion and/or said powder aggregate within a mold, die, other cavity or other shape-forming means.

7. The process of any preceding claim, wherein said RF energy has a frequency of from 1 MHz to 300 MHz, preferably from 1 MHz to 100 MHz, more preferably from 10 MHz to 50 MHz , and most preferably 24.4 MHz, 27.12 MHz, 13.56 MHz or 40.68MHz.

8. The process of any preceding claim, wherein said gum base is chosen among one or more of any conventional gum base known in the art including gum base of natural or synthetic origin, whereby gum bases of natural origin include, but are not limited to, chicle, jelutong-, lechi de caspi-, soh-, siak-, katiau-, sorwa-, balata-, pendare-, malaya-, and peach gums; natural cautchouc agar, alginate, Arabic gum, carob gum, carrageenan, ghatti gum, guar gum, karaya gum, pectin, tragacanth, locust bean gum, gellan gum and xanthan gum; and natural resins such as dammar and mastix, and gum bases of synthetic origin may be mixtures of elastomers (polymers, masticating substances), plasticizer (resin, elastomers, solvent, hydrophobic resin), filler (texturizer, water-insoluble adjuvant), softener (fat), emulsifier, wax, antioxidant, and anti-tacking agents (vinyl polymer hydrophilic resin).

9. The process of any preceding claim, wherein the weight percentage of gum base in the gum-base comprising powder is from 10% to 80%, preferably from 20% to 80 %, more preferably from 30% to 80%, and even more preferably from 40% to 70%.

10. The process of any preceding claim, wherein said gum-base comprising powder comprises nicotine in any form.

11. The process of claim 2-10, wherein said one or more deposits comprises nicotine in any form.

12. The process of claim 10-11, wherein nicotine in any form is chosen from the group consisting of a nicotine salt, the free base form of nicotine, a nicotine derivative, such as a nicotine cation exchanger, a nicotine inclusion complex such as cyclodextrin complex, or nicotine in any non-covalent binding, nicotine bound to zeolites, and nicotine bound to cellulose including micro-crystalline cellulose, or starch microspheres.

13. The process of claim 12, wherein the nicotine cation exchanger is a polyacrylate cation exchanger such as, but not limited to, Amberlite IRC 50 (Rohm & Haas), Amberlite IRP 64 (Rohm & Haas), Amberlite IRP 64M (Rohm & Haas), BIO-REX 70 (BIO-RAD Lab.), Amberlite IR 118 (Rohm & Haas), Amberlite IRP 69 (Rohm & Haas), Amberlite IRP 69M (Rohm & Haas), BIO-REX 40 (BIO-RAD Lab.), Amberlite IR 120 (Rohm & Haas), Dowex 50 (Dow Chemical), Dowex 50W (Dow Chemical), Duolite C 25 (Chemical Process Co.), Lewatit S 100 (Farbenfabriken Bayer), Ionac C 240 (Ionac Chem.), Wofatit KP S 200 (I.G. Farben Wolfen), Amberlyst 15 (Rohm & Haas), Duolite C-3 (Chemical Process), Duolite C-10 (Chemical Process), Lewatit KS (Farbenfabriken Bayer), Zerolit 215 (The Permutit Co.), Duolite ES-62 (Chemical Process), BIO-REX 63 (BIO-RAD Lab.), Duolite ES-63 (Chemical Process), Duolite ES-65 (Chemical Process), Ohelex 100 (BIO-RAD Lab.), Dow Chelating Resin A-1 (Dow Chemical Company), Purolite C115HMR (Purolite International Ltd.), CM Sephadex C-25 (Pharmacia Fine Chemicals), SE Sephadex C-25 (Pharmacia Fine Chemicals), Viscarin GP-109NF Lambda-carrageenan FMC Biopolymer or any other anionic polyelectrolyte.

14. The process of claim 12, wherein the nicotine salt may be, but is not limited to, mono-tartrate, hydrogen tartrate, citrate, malate, hydrochloride, and formic, acetic, propionic, butyric, 2-methylbutyric, 3-methylbutynic, valeric, lauric, palmitic, oxalic, benzoic, gentisic, gallic, phenylacetic, salicylic, phthalic, picric, sulfosalicylic, tannic, pectic, alginic, chloroplatinic, silcotungstic, pyruvic, glutamic, and aspartic salt of nicotine.

15. The process of claim 11-14, wherein nicotine in any form is present in an amount of from 0.05 mg to 12 mg calculated as the free base form of nicotine per chewing gum, preferably in an amount of from 0.2 mg to 8 mg, more preferably in an amount of from 0.5 mg to 6 mg, even more preferably in an amount from 1 mg to 5 mg.

16. The process of any preceding claim, wherein said gum-base-comprising powder and/or said deposit(s) further comprises one or more additional ingredients, preferably one or more buffers, one or more softeners, one or more thickening agents, one or more fillers, one or more emulsifiers, one or more glidants, one or more lubricants, one or more sweeteners, one or more flavors, one or more aromatics, one or more enhancers, one or more coloring agents, one or more preservatives, and/or mixtures thereof.

17. The process of claim 16, wherein one or more of the additional ingredients is present in encapsulated form and/or as flakes or part of flakes and/or fracture sensitive formats.

18. The process of any preceding claim, wherein the amount of buffering agent or agents in the chewing gum is present in an amount sufficient to raise the pH of the saliva in the oral cavity of a subject to above 7 and to transiently maintain the pH of the saliva in the oral cavity above 7.

19. The process of any preceding claim, wherein said process comprises the steps of:
i. dispensing a powder portion from a gum-base-comprising powder,
ii. shaping said powder portion into a powder aggregate in a die by volume reducing said powder aggregate by introducing at least one punch into said die thereby applying sufficient force,
iii. applying sufficient radio frequency (RF) energy to said powder aggregate to transform said powder aggregate into said chewing gum
iv. and removing said chewing gum from said die.

20. The process of claim 19, wherein said process further comprises the step of cooling said chewing gum in said die prior to removing said chewing gum from said die.

21. The process of claim 19, wherein said at least one punch comprises an electrode, which delivers said RF energy to said powder aggregate.

22. The process of claim 19, wherein said die comprises an electrode, which delivers said RF energy to said powder aggregate.

23. The process of claim 19, wherein said gum-base comprising powder is densified using an upper punch and a lower punch, and at least one of said upper punch or lower punch comprises an electrode, which delivers said RF energy to said powder aggregate.

24. The process of claim 1, wherein said process further comprises coating said chewing gum.

25. The process of any preceding claim, wherein at least one of forming, making and adhering of one or more of said one or more deposit(s) takes place concomitantly with the processing of the gum base-comprising powder.

26. The process of any preceding claim, wherein at least one of forming, making and adhering of one or more of said one or more deposit(s) take(s) place separately from the processing of the gum base-comprising powder.

27. The process of any of claims 25 - 26, wherein at least one of said one or more deposit(s) is made using application of RF energy.

28. The process of any of claims 25 - 27, wherein at least one of said one or more deposit(s) is made using any of compression, compaction, slugging, coating, molding, extrusion and/or granulation

29. The process of any of claims 25 - 28, wherein the adhering of said one or more deposit(s) is achieved by application of RF energy.

30. The process of any of claims 21 - 29, wherein incompatible ingredients are separated from each other by being located in separate parts of the chewing gum.

31. The process of any of claims 25 - 30, wherein one or more deposits are located at least partly at the peripheral part of the chewing gum.

32. The process of any of claims 25 - 31, wherein one or more deposits are located at least partly within the chewing gum.

33. The process of any preceding claim, wherein the chewing gum essentially has the form of a parallelepiped, a three-dimensional representation of a spinnaker shape, a crescent, a hamburger, a disc, a heart, a polygon, a hexaflexagon, a circular object, an oval object, an oblong object, a polyhedron, such as a cube, a pyramid, a prism, a triangle, or the like; a space figure with some non-flat faces, such as a cone, a truncated cone, a cylinder, a sphere, a capsule-shaped object, a torus, or the like, whereby the chewing gum optionally has one or more major faces.

34. The process of any preceding claim, wherein the gum-base comprising powder is a blend of powders with different properties.

35. The process of any preceding claim, wherein the one or more deposits are different between themselves.

36. The process of any preceding claim, where the chewing gum is further treated with EM energy, preferably RF energy, and/or thermal and/or mechanical energy.

## Patentansprüche

1. Verfahren zur Herstellung eines nikotinhaltigen Kaugummis, wobei das Verfahren die folgenden Schritte umfasst
i. Abgeben eines Pulveranteils aus einem gummibasehaltigen Pulver,
ii. wahlweise Formen des Pulveranteils zu einem Pulveraggregat,
iii. und Anlegen einer ausreichenden elektromagnetischen Energie (EM-Energie) an den Pulveranteil oder das Pulveraggregat, um den Pulveranteil oder das Pulveraggregat in den nikotinhaltigen Kaugummi umzuwandeln,
wobei die EM-Energie Radiofrequenz(RF)-Energie, MicroWave(MW)-Energie, Infrarot(IR)-Energie oder Ultraviolett(UV)-Energie oder Kombinationen davon ist, vorzugsweise Radiofrequenz(RF)-Energie, die Kombination aus RF-Energie und IR-Energie, die Kombination aus RF-Energie und MW-Energie und die Kombination aus RF-Energie, IR-Energie und MW-Energie.

2. Verfahren nach Anspruch 1, wobei der Kaugummi ferner eine oder mehrere Ablagerungen umfasst, wobei solche Ablagerungen vorzugsweise, aber nicht ausschließlich, ausgewählt sind aus Schichten, Filmen, Beschichtungen wie Zuckerbeschichtungen, Filmbeschichtungen, Pressbeschichtungen, Kompressionsbeschichtungen und Schmelzbeschichtungen, Perlen, Tabletten, Kapseln, Flocken, Granalien, Pillen, Pastillen, hartgekochten Lutschtabletten, Geleegummis und Gelen und/oder Kombinationen davon, wobei die Ablagerungen wahlweise anfangs ein Pulver umfassen können, wobei eine oder mehrere Ablagerungen auf der Außenseite des Kaugummis (äußere Ablagerung) angeordnet sein können oder eine oder mehrere Ablagerungen innerhalb des Kaugummis (innere Ablagerung) angeordnet sein können oder mindestens eine Ablagerung eine äußere Ablagerung und mindestens eine Ablagerung eine innere Ablagerung ist.

3. Verfahren nach Anspruch 1, wobei der Pulveranteil in ein Formteil, eine Form, einen anderen Hohlraum oder andere formbildende Mittel abgegeben wird.

4. Verfahren nach einem der vorhergehenden Ansprüche, wobei das wahlweise Formen des Pulveranteils zu einem Pulveraggregat eine Verdichtung wie Stampfen, Verdichten, Kompaktieren, Entlüften, Vakuumformen, Schleudern, Granulieren und Vibrieren umfasst.

5. Verfahren nach einem der Ansprüche 1 bis 4, wobei die Umwandlung des Pulveranteils und/oder des Pulveraggregats zu einem Kaugummi durch Sintern und/oder Vereinigen und/oder Schmelzen und/oder mechanisches Ineinandergreifen erhalten wird.

6. Verfahren nach einem der Ansprüche 1 bis 5, wobei die RF-Energie an den Pulveranteil und/oder das Pulveraggregat in einem Formteil, einer Form, einem anderen Hohlraum oder anderen formgebenden Mitteln angelegt wird.

7. Verfahren nach einem der vorhergehenden Ansprüche, wobei die RF-Energie eine Frequenz von 1 MHz bis 300 MHz, vorzugsweise von 1 MHz bis 100 MHz, mehr bevorzugt von 10 MHz bis 50 MHz und am meisten bevorzugt von 24,4 MHz, 27,12 MHz, 13,56 MHz oder 40,68 MHz aufweist.

8. Verfahren nach einem der vorhergehenden Ansprüche, wobei die Gummibase aus einer oder mehreren herkömmlichen Gummibasen ausgewählt ist, die im Stand der Technik bekannt sind, einschließlich Gummibase natürlichen oder synthetischen Ursprungs, wobei die Gummibasen natürlichen Ursprungs Chicle, Jelutong-, Lechi de caspi-, Soh-, Siak-, Katiau-, Sorwa-, Balata-, Pendare-, Malaya- und Pfirsichgummi; Naturkautschuk-Agar, Alginat, arabisches Gummi, Johannisbrotgummi, Carrageenan, Ghatti-Gummi, Guargummi, Karayagummi, Pektin, Traganth, Locust-Bohnengummi, Gellangummi und Xanthangummi; und natürliche Harze wie Dammar und Mastix einschließen, aber nicht darauf beschränkt sind, und Gummibasen synthetischen Ursprungs Mischungen von Elastomeren (Polymere, Mastiziersubstanzen), Weichmacher (Harz, Elastomere, Lösungsmittel, hydrophobes Harz), Füllstoff (Texturbildner, wasserunlösliches Adjuvans), Erweichungsmittel (Fett), Emulgator, Wachs, Antioxidationsmittel und Antiklebmittel (hydrophiles Vinylpolymerharz) sein können.

9. Verfahren nach einem der vorhergehenden Ansprüche, wobei der Gewichtsprozentanteil der Gummibase in dem gummibasehaltigen Pulver 10% bis 80%, vorzugsweise 20% bis 80%, mehr bevorzugt 30% bis 80% und noch mehr bevorzugt von 40% bis 70% beträgt.

10. Verfahren nach einem der vorhergehenden Ansprüche, wobei das gummibasehaltige Pulver Nikotin in jeder beliebigen Form umfasst.

11. Verfahren nach Anspruch 2 bis 10, wobei die eine oder die mehreren Ablagerungen Nikotin in jeder beliebigen Form umfassen.

12. Verfahren nach Anspruch 10 bis 11, wobei Nikotin in einer beliebigen Form ausgewählt ist aus der Gruppe, bestehend aus einem Nikotinsalz, der freien Basenform von Nikotin, einem Nikotinderivat, wie einem Nikotin-Kationenaustauscher, einem Nikotin-Einschlusskomplex wie Cyclodextrinkomplex, oder Nikotin in jeder nichtkovalenten Bindung, an Zeolithe gebundenes Nikotin und an Cellulose gebundenes Nikotin, einschließlich mikrokristalliner Cellulose, oder Stärke-Mikrokügelchen.

13. Verfahren nach Anspruch 12, wobei der Nikotin-Kationenaustauscher ein Polyacrylat-Kationenaustauscher ist wie Amberlite IRC 50 (Rohm & Haas), Amberlite IRP 64 (Rohm & Haas), Amberlite IRP 64M (Rohm & Haas), BIO-REX 70 (BIO-RAD Lab.), Amberlite IR 118 (Rohm & Haas), Amberlite IRP 69 (Rohm & Haas), Amberlite IRP 69M (Rohm & Haas), BIO-REX 40 (BIO-RAD Lab.), Amberlite IR 120 (Rohm & Haas), Dowex 50 (Dow Chemical), Dowex 50W (Dow Chemical), Duolite C 25 (Chemical Process Co.), Lewatit S 100 (Farbenfabriken Bayer), Ionac C 240 (Ionac Chem.), Wofatit KP S 200 (I.G. Farben Wolfen), Amberlyst 15 (Rohm & Haas), Duo lite C-3 (Chemical Process), Duo lite C-10 (Chemical Process), Lewatit KS (Farbenfabriken Bayer), Zerolit 215 (The Permutit Co.), Duolite ES-62 (Chemical Process), BIO-REX 63 (BIO-RAD Lab.), Duolite ES-63 (Chemical Process), Duolite ES-65 (Chemical Process), Ohelex 100 (BIO-RAD Lab.), Dow Chelating Resin A-1 (Dow Chemical Company), Purolite C115HMR (Purolite International Ltd.), CM Sephadex C-25 (Pharmacia Fine Chemicals), SE Sephadex C-25 (Pharmacia Fine Chemicals), Viscarin GP-109NF Lambda-carrageenan FMC Biopolymer oder jedes andere anionische Polyelektrolyt.

14. Verfahren nach Anspruch 12, wobei das Nikotinsalz Mono-Tartrat, Hydrogentartrat, Citrat, Malat, Hydrochlorid und Ameisensäure, Essigsäure, Propionsäure, Buttersäure, 2-Methylbuttersäure, 3-Methylbutinsäure, Valeriansäure, Laurinsäure, Palmitinsäure, Oxalsäure, Benzoesäure, Gentisinsäure, Gallsäure, Phenylessigsäure, Salicylsäure, Phthalsäure, Picrinsäure, Sulfosalicylsäure, Gerbsäure, Pectinsäure, Alginsäure, Chlorplatinsäure, Silcowolframsäure, Brenztraubensäure, Glutaminsäure und Asparaginsalz von Nikotin sein kann, aber nicht darauf beschränkt ist.

15. Verfahren nach Anspruch 11 bis 14, wobei Nikotin in jeder beliebigen Form in einer Menge von 0,05 mg bis 12 mg vorliegt, berechnet als die freie Basenform von Nikotin pro Kaugummi, vorzugsweise in einer Menge von 0,2 mg bis 8 mg, mehr bevorzugt in einer Menge von 0,5 mg bis 6 mg, noch bevorzugter in einer Menge von 1 mg bis 5 mg.

16. Verfahren nach einem der vorhergehenden Ansprüche, wobei das gummibasenhaltige Pulver und/oder die Ablagerung(en) ferner einen oder mehrere zusätzliche Bestandteile, vorzugsweise einen oder mehrere Puffer, einen oder mehrere Erweichungsmittel, eines oder mehrere Verdickungsmittel, einen oder mehrere Füllstoffe, einen oder mehrere Emulgatoren, eines oder mehrere Gleitmittel, eines oder mehrere Schmiermittel, einen oder mehrere Süßstoffe, einen oder mehrere Geschmacksstoffe, eines oder mehrere Aromaten, einen oder mehrere Geschmacksverstärker, einen oder mehrere Farbstoffe, eines oder mehrere Konservierungsmittel und/oder Mischungen davon umfasst.

17. Verfahren nach Anspruch 16, wobei einer oder mehrere der zusätzlichen Bestandteile in verkapselter Form und/oder als Flocken oder Teil von Flocken und/oder zerbrechlichen Formaten vorliegen.

18. Verfahren nach einem der vorhergehenden Ansprüche, wobei die Menge des Puffermittels oder der Puffermittel in dem Kaugummi in einer Menge vorhanden ist, die ausreicht, um den pH-Wert des Speichels in der Mundhöhle eines Subjekts auf über 7 zu erhöhen und den pH-Wert des Speichels in der Mundhöhle vorübergehend über 7 zu halten.

19. Verfahren nach einem der vorhergehenden Ansprüche, wobei das Verfahren die folgenden Schritte umfasst:
i. Abgeben eines Pulveranteils aus einem gummibasehaltigen Pulver,
ii. Formen des Pulveranteils zu einem Pulveraggregat in einer Form durch Volumenreduzierung des Pulveraggregats durch Einführen mindestens eines Stempels in die Form, wodurch eine ausreichende Kraft angelegt wird,
iii. Anlegen von ausreichender Radiofrequenz(RF) - Energie an das Pulveraggregat, um das Pulveraggregat in den Kaugummi umzuwandeln,
iv. und Entfernen des Kaugummis aus der Form.

20. Verfahren nach Anspruch 19, wobei das Verfahren ferner den Schritt des Abkühlens des Kaugummis in der Form vor dem Entfernen des Kaugummis aus der Form umfasst.

21. Verfahren nach Anspruch 19, wobei der mindestens eine Stempel eine Elektrode umfasst, welche die RF-Energie an das Pulveraggregat abgibt.

22. Verfahren nach Anspruch 19, wobei die Form eine Elektrode umfasst, welche die RF-Energie an das Pulveraggregat abgibt.

23. Verfahren nach Anspruch 19, wobei gummibasehaltige Pulver unter Verwendung eines oberen Stempels und eines unteren Stempels verdichtet wird und mindestens einer von dem oberen Stempel oder unteren Stempel eine Elektrode umfasst, welche die RF-Energie an das Pulveraggregat abgibt.

24. Verfahren nach Anspruch 1, wobei das Verfahren ferner das Beschichten des Kaugummis umfasst.

25. Verfahren nach einem der vorhergehenden Ansprüche, wobei mindestens eines von Formen, Herstellen und Anhaften einer oder mehrerer der einen oder mehreren Ablagerungen gleichzeitig mit der Verarbeitung des gummibasenhaltigen Pulvers erfolgt.

26. Verfahren nach einem der vorhergehenden Ansprüche, wobei mindestens eines von Formen, Herstellen und Anhaften einer oder mehrerer der einen oder mehreren Ablagerungen separat von der Verarbeitung des gummibasenhaltigen Pulvers erfolgt.

27. Verfahren nach einem der Ansprüche 25 bis 26, wobei mindestens eine der einen oder mehreren Ablagerungen durch Anlegen von RF-Energie hergestellt wird.

28. Verfahren nach einem der Ansprüche 25 bis 27, wobei mindestens eine der einen oder mehreren Ablagerungen unter Verwendung eines beliebigen von Kompression, Kompaktierung, Schleudern, Beschichten, Gießen, Extrusion und/oder Granulieren hergestellt wird.

29. Verfahren nach einem der Ansprüche 25 bis 28, wobei das Anhaften einer der einen oder mehreren Ablagerungen durch Anlegen von RF-Energie erreicht wird.

30. Verfahren nach einem der Ansprüche 21 bis 29, wobei inkompatible Bestandteile voneinander getrennt werden, indem sie in separaten Teilen des Kaugummis angeordnet werden.

31. Verfahren nach einem der Ansprüche 25 bis 30, wobei eine oder mehrere Ablagerungen mindestens teilweise im Teil des Umfangs des Kaugummis angeordnet sind.

32. Verfahren nach einem der Ansprüche 25 bis 31, wobei eine oder mehrere Ablagerungen mindestens teilweise innerhalb des Kaugummis angeordnet sind.

33. Verfahren nach einem der vorhergehenden Ansprüche, wobei der Kaugummi im Wesentlichen die Form eines Parallelepipeds, einer dreidimensionalen Darstellung einer Spinnakerform, eines Halbmondes, eines Hamburgers, einer Scheibe, eines Herzens, eines Vielecks, eines Hexaflexagons, eines kreisförmigen Objekts, eines ovalen Objekts, eines länglichen Objekts, eines Polyeders, wie eines Würfels, einer Pyramide, eines Prismas, eines Dreiecks oder dergleichen; einer räumlichen Figur mit einigen nicht flachen Flächen, wie beispielsweise eines Kegels, eines Kegelstumpfes, eines Zylinders, einer Kugel, eines kapselförmigen Objekts, eines Torus oder dergleichen aufweist, wobei der Kaugummi wahlweise eine oder mehrere Hauptflächen aufweist.

34. Verfahren nach einem der vorhergehenden Ansprüche, wobei das gummibasehaltige Pulver eine Mischung von Pulvern mit unterschiedlichen Eigenschaften ist.

35. Verfahren nach einem der vorhergehenden Ansprüche, wobei die eine oder die mehreren Ablagerungen voneinander unterschiedlich sind.

36. Verfahren nach einem der vorhergehenden Ansprüche, wobei der Kaugummi ferner mit EM-Energie, vorzugsweise RF-Energie und/oder thermischer und/oder mechanischer Energie behandelt wird.

## Revendications

1. Procédé de fabrication d'un chewing-gum comprenant de la nicotine, ledit procédé comprenant les étapes consistant à :
i. distribuer une portion de poudre à partir d'une poudre comprenant une base de gomme,
ii. éventuellement façonner ladite portion de poudre en un agrégat de poudre,
iii. et appliquer une énergie électromagnétique suffisante (énergie EM) à ladite portion de poudre ou audit agrégat de poudre afin de transformer ladite portion de poudre ou ledit agrégat de poudre en ledit chewing-gum comprenant de la nicotine,
où ladite énergie EM est une énergie de Radiofréquence (RF), une énergie de Microondes (MW), une énergie d'Infrarouge (IR) ou une énergie d'Ultraviolet (UV) ou des combinaisons de celles-ci, préférablement une énergie de Radiofréquence (RF), la combinaison d'énergie RF et d'énergie IR, la combinaison d'énergie RF et d'énergie MW, et la combinaison d'énergie RF, d'énergie IR et d'énergie MW.

2. Procédé selon la revendication 1, dans lequel ledit chewing-gum comprend en outre un ou plusieurs dépôts, de tels dépôts étant choisis de préférence, mais pas exclusivement, parmi les couches, les films, les enrobages, tels que les enrobages en sucre, les enrobages en film, les enrobages à sec, les enrobages par compression, et les enrobages à l'état fondu, les billes, les comprimés, les capsules, les flocons, les granulés, les pilules, les pastilles, les bonbons durs, les bonbons gélifiés et les gels et/ou des combinaisons de ceux-ci, où éventuellement lesdits dépôts peuvent initialement comprendre de la poudre, où un ou plusieurs dépôts peuvent être placés à l'extérieur du chewing-gum (dépôt extérieur) ou un ou plusieurs dépôts peuvent être placés au sein du chewing-gum (dépôt intérieur) ou au moins un dépôt est un dépôt extérieur et au moins un dépôt est un dépôt intérieur.

3. Procédé selon la revendication 1, dans lequel ladite portion de poudre est distribuée sur un moule, une filière, une autre cavité ou un autre moyen de mise en forme.

4. Procédé selon l'une quelconque des revendications précédentes, dans lequel ledit façonnage facultatif de ladite portion de poudre en un agrégat de poudre comprend une densification, telle qu'un(e) ou plusieurs parmi le damage, la compression, la compaction, la désaération, la formation sous vide, le briquetage, la granulation et la vibration.

5. Procédé selon l'une quelconque des revendications 1-4, dans lequel la transformation de la portion de poudre et/ou de l'agrégat de poudre en un chewing-gum est obtenue par frittage et/ou fonte et/ou fusion et/ou emboîtement mécanique.

6. Procédé selon l'une quelconque des revendications 1-5, dans lequel ladite énergie RF est appliquée à ladite portion de poudre et/ou audit agrégat de poudre au sein d'un moule, d'une filière, d'une autre cavité ou d'un autre moyen de mise en forme.

7. Procédé selon l'une quelconque des revendications précédentes, dans lequel ladite énergie RF possède une fréquence allant de 1 MHz à 300 MHz, préférablement de 1 MHz à 100 MHz, plus préférablement de 10 MHz à 50 MHz, et tout préférablement de 24,4 MHz, 27,12 MHz, 13,56 MHz ou 40,68 MHz.

8. Procédé selon l'une quelconque des revendications précédentes, dans lequel ladite base de gomme est choisie parmi une ou plusieurs parmi de quelconques bases de gomme classiques connues dans l'état de la technique y compris des bases de gomme d'origine naturelle ou synthétique, où les bases de gomme d'origine naturelle comportent, sans y être limitées, les gommes de chicle, de jelutong, de lechi-caspi, de soh, de siak, de katiau, de sorwa, de balata, de pendare, de malaya, et de pêche ; le caoutchouc naturel, l'agar-agar, l'alginate, la gomme arabique, la gomme du caroubier, le carraghénane, la gomme ghatti, la gomme guar, la gomme karaya, la pectine, la gomme adragante, la gomme de caroube, la gomme gellane et la gomme xanthane ; et les résines naturelles telles que le dammar et le mastic, et les bases de gomme d'origine synthétique peuvent être des mélanges d'élastomères (polymères, substances de mastication), de plastifiants (résine, élastomères, solvant, résine hydrophobe), de charges (agent de texture, adjuvant insoluble dans l'eau), d'adoucissant (matière grasse), d'émulsifiant, de cire, d'antioxydant, et d'agents anti-poisseux (résine hydrophile à base de polymère vinylique).

9. Procédé selon l'une quelconque des revendications précédentes, dans lequel le pourcentage pondéral de la base de gomme dans la poudre comprenant une base de gomme va de 10% à 80%, préférablement de 20% à 80%, plus préférablement de 30% à 80%, et encore plus préférablement de 40% à 70%.

10. Procédé selon l'une quelconque des revendications précédentes, dans lequel ladite poudre comprenant une base de gomme comprend de la nicotine sous une forme quelconque.

11. Procédé selon les revendications 2-10, dans lequel lesdits un ou plusieurs dépôts comprennent de la nicotine sous une forme quelconque.

12. Procédé selon les revendications 10-11, dans lequel la nicotine sous une forme quelconque est choisie dans le groupe constitué par un sel de nicotine, la forme de base libre de nicotine, un dérivé de nicotine, tel qu'un échangeur de cations de nicotine, un complexe d'inclusion de nicotine tel qu'un complexe de cyclodextrine, ou de la nicotine dans une liaison non covalente quelconque, de la nicotine liée à des zéolithes, et de la nicotine liée à de la cellulose y compris de la cellulose microcristalline, ou des microsphères d'amidon.

13. Procédé selon la revendication 12, dans lequel l'échangeur de cations de nicotine est un échangeur de cations à base de polyacrylate tel que, dans y être limité, Amberlite IRC 50 (Rohm & Haas), Amberlite IRP 64 (Rohm & Haas), Amberlite IRP 64M (Rohm & Haas), BIO-REX 70 (BIO-RAD Lab.), Amberlite IR 118 (Rohm & Haas), Amberlite IRP 69 (Rohm & Haas), Amberlite IRP 69M (Rohm & Haas), BIO-REX 40 (BIO-RAD Lab.), Amberlite IR 120 (Rohm & Haas), Dowex 50 (Dow Chemical), Dowex 50W (Dow Chemical), Duolite C 25 (Chemical Process Co.), Lewatit S 100 (Farbenfabriken Bayer), Ionac C 240 (Ionac Chem.), Wofatit KP S 200 (I.G. Farben Wolfen), Amberlyst 15 (Rohm & Haas), Duolite C-3 (Chemical Process), Duolite C-10 (Chemical Process), Lewatit KS (Farbenfabriken Bayer), Zerolit 215 (The Permutit Co.), Duolite ES-62 (Chemical Process), BIO-REX 63 (BIO-RAD Lab.), Duolite ES-63 (Chemical Process), Duolite ES-65 (Chemical Process), Ohelex 100 (BIO-RAD Lab.), Dow Chelating Resin A-1 (Dow Chemical Company), Purolite C115HMR (Purolite International Ltd.), CM Sephadex C-25 (Pharmacia Fine Chemicals), SE Sephadex C-25 (Pharmacia Fine Chemicals), le lambda-carraghénane Viscarin GP-109NF de FMC Biopolymer ou tout autre polyélectrolyte anionique.

14. Procédé selon la revendication 12, dans lequel le sel de nicotine peut être, sans y être limité, un sel de monotartrate, hydrogénotartrate, citrate, malate, chlorhydrate, et formique, acétique, propionique, butyrique, 2-méthylbutyrique, 3-méthylbutynique, valérique, laurique, palmitique, oxalique, benzoïque, gentisique, gallique, phénylacétique, salicylique, phtalique, picrique, sulfosalicylique, tannique, pectique, alginique, chloroplatinique, silcotungstique, pyruvique, glutamique, et aspartique de nicotine.

15. Procédé selon les revendications 11-14, dans lequel la nicotine sous une forme quelconque est présente selon une quantité allant de 0,05 mg à 12 mg calculée en tant que forme de base libre de la nicotine par chewing-gum, préférablement selon une quantité allant de 0,2 mg à 8 mg, plus préférablement selon une quantité allant de 0,5 mg à 6 mg, encore plus préférablement selon une quantité allant de 1 mg à 5 mg.

16. Procédé selon l'une quelconque des revendications précédentes, dans lequel ladite poudre comprenant une base de gomme et/ou ledit ou lesdits dépôts comprennent en outre un ou plusieurs ingrédients supplémentaires, préférablement un ou plusieurs tampons, un ou plusieurs adoucissants, un ou plusieurs agents épaississants, une ou plusieurs charges, un ou plusieurs émulsifiants, un ou plusieurs agents de glissement, un ou plusieurs lubrifiants, un ou plusieurs édulcorants, un ou plusieurs arômes, une ou plusieurs substances aromatiques, un ou plusieurs exhausteurs, un ou plusieurs agents colorants, un ou plusieurs conservateurs, et/ou des mélanges de ceux-ci.

17. Procédé selon la revendication 16, dans lequel un ou plusieurs parmi les ingrédients supplémentaires est présent sous forme encapsulée et/ou sous forme de flocons ou partie de flocons et/ou de formats sensibles à la fissuration.

18. Procédé selon l'une quelconque des revendications précédentes, dans lequel la quantité d'agents tampon ou d'agents dans le chewing-gum est présente selon une quantité suffisante pour augmenter le pH de la salive dans la cavité orale d'un sujet jusqu'à plus de 7 et de maintenir de manière transitoire le pH de la salive dans la cavité orale à plus de 7.

19. Procédé selon l'une quelconque des revendications précédentes, où ledit procédé comprend les étapes consistant à :
i. distribuer une portion de poudre à partir d'une poudre comprenant une base de gomme,
ii. façonner ladite portion de poudre en un agrégat de poudre dans une filière par une réduction de volume dudit agrégat de poudre par l'introduction d'au moins un poinçon dans ladite filière, appliquant ainsi une force suffisante,
iii. appliquer une énergie de radiofréquence (RF) suffisante audit agrégat de poudre afin de transformer ledit agrégat de poudre en ledit chewing-gum,
iv. et retirer ledit chewing-gum de ladite filière.

20. Procédé selon la revendication 19, où ledit procédé comprend en outre l'étape consistant à refroidir ledit chewing-gum dans ladite filière préalablement au retrait dudit chewing-gum de ladite filière.

21. Procédé selon la revendication 19, dans lequel ledit au moins un poinçon comprend une électrode, qui distribue ladite énergie RF audit agrégat de poudre.

22. Procédé selon la revendication 19, dans lequel ladite filière comprend une électrode, qui distribue ladite énergie RF audit agrégat de poudre.

23. Procédé selon la revendication 19, dans lequel ladite poudre comprenant une base de gomme est densifiée en utilisant un poinçon supérieur et un poinçon inférieur, et au moins l'un parmi ledit poinçon supérieur ou poinçon inférieur comprend une électrode, qui distribue ladite énergie RF audit agrégat de poudre.

24. Procédé selon la revendication 1, où ledit procédé comprend en outre l'enrobage dudit chewing-gum.

25. Procédé selon l'une quelconque des revendications précédentes, dans lequel au moins un(e) parmi le façonnage, la préparation et l'adhésion d'un ou plusieurs parmi lesdits un ou plusieurs dépôts a lieu de manière concomitante avec la transformation de la poudre comprenant une base de gomme.

26. Procédé selon l'une quelconque des revendications précédentes, dans lequel au moins un(e) parmi le façonnage, la préparation et l'adhésion d'un ou plusieurs parmi lesdits un ou plusieurs dépôts a lieu séparément de la transformation de la poudre comprenant une base de gomme.

27. Procédé selon l'une quelconque des revendications 25-26, dans lequel au moins l'un parmi lesdits un ou plusieurs dépôts est préparé en utilisant l'application d'énergie RF.

28. Procédé selon l'une quelconque des revendications 25-27, dans lequel au moins l'un parmi lesdits un ou plusieurs dépôts est préparé en utilisant l'un(e) quelconque parmi la compression, la compaction, le briquetage, l'enrobage, le moulage, l'extrusion et/ou la granulation.

29. Procédé selon l'une quelconque des revendications 25-28, dans lequel l'adhésion desdits un ou plusieurs dépôts est obtenue par l'application d'énergie RF.

30. Procédé selon l'une quelconque des revendications 21-29, dans lequel les ingrédients incompatibles sont séparés les uns des autres en étant situés dans des paries séparées du chewing-gum.

31. Procédé selon l'une quelconque des revendications 25-30, dans lequel un ou plusieurs dépôts sont situés au moins en partie au niveau de la partie périphérique du chewing-gum.

32. Procédé selon l'une quelconque des revendications 25-31, dans lequel un ou plusieurs dépôts sont situés au moins en partie au sein du chewing-gum.

33. Procédé selon l'une quelconque des revendications précédentes, dans lequel le chewing-gum possède essentiellement la forme d'un parallélépipède, une représentation tridimensionnelle d'une forme de spinnaker, un croissant, un hamburger, un disque, un coeur, un polygone, un hexaflexagone, un objet circulaire, un objet ovale, un objet oblong, un polyèdre, tel qu'un cube, une pyramide, un prisme, un triangle, ou similaire ; une figure spatiale ayant quelques faces non plates, telle qu'un cône, un cône tronqué, un cylindre, une sphère, un objet en forme de capsule, un tore, ou similaire, où le chewing-gum possède éventuellement une ou plusieurs faces principales.

34. Procédé selon l'une quelconque des revendications précédentes, dans lequel la poudre comprenant une base de gomme est un assemblage de poudres ayant différentes propriétés.

35. Procédé selon l'une quelconque des revendications précédentes, dans lequel les un ou plusieurs dépôts sont différents entre eux.

36. Procédé selon l'une quelconque des revendications précédentes, dans lequel le chewing-gum est traité davantage par une énergie EM, préférablement une énergie RF, et/ou une énergie thermique et/ou mécanique.
